# EUROPEAN PATENT SPECIFICATION

(11) **EP 3 368 088 B1**
(45) Date of publication and mention of the grant of the patent: **07.06.2023**
(21) Application number: 16791282.3
(22) Date of filing: 31.10.2016
(51) Int. Cl.: A61K 47/60, A61K 47/59, A61K 47/50, A61K 49/00, A61K 31/05, A61K 31/192, A61K 31/195, A61K 31/573, A61P 43/00, A61P 39/06, A61P 25/00, A61P 15/08, A61P 3/06, A61P 25/28, A61P 17/18

(54) **COMPOSITIONS AND METHODS FOR TREATMENT OF PEROXISOMAL DISORDERS AND LEUKODYSTROPHIES**
ZUSAMMENSETZUNGEN UND VERFAHREN ZUR BEHANDLUNG VON PEROXISOMALEN ERKRANKUNGEN UND LEUKODYSTROPHIEN
COMPOSITIONS ET MÉTHODES POUR LE TRAITEMENT DE TROUBLES DU PEROXISOME ET DE LEUCODYSTROPHIES

(30) Priority: 29.10.2015 US 201562248163 P
(43) Date of publication of application: 05.09.2018
(73) Proprietor: The Johns Hopkins University, Baltimore, MD 21218 (US); Kennedy Krieger Institute, Inc., Baltimore, Maryland 21205 (US)
(72) Inventor: KANNAN, Sujatha, Highland, MD 20777 (US); RANGARAMANUJAM, Kannan, Highland, MD 20777 (US); ZHANG, Fan, Baltimore, MD 21218 (US); FATEMI, Seyed Ali, Bethesda, MD 20814 (US); TURK, Bela, 1050 Vienna (AT); GOK, Ozgul, Istanbul (TR)
(74) Representative: Potter Clarkson
(86) International application number: PCT/US2016/059697
(87) International publication number: WO 2017/075580

(56) References cited:
- WO-A1-00/18394
- WO-A1-2009/142754
- WO-A1-2014/178892
- WO-A1-2014/197909
- WO-A1-2015/027068
- WO-A1-2016/025745
- WO-A2-2009/046446
- US-A1- 2008 031 848
- US-A1- 2012 003 155
- MANOJ K. MISHRA ET AL: "Dendrimer Brain Uptake and Targeted Therapy for Brain Injury in a Large Animal Model of Hypothermic Circulatory Arrest", ACS NANO, vol. 8, no. 3, 25 March 2014 (2014-03-25), pages 2134-2147, XP55235336, US ISSN: 1936-0851, DOI: 10.1021/nn404872e
- ELIZABETH NANCE ET AL: "Systemic dendrimer-drug treatment of ischemia-induced neonatal white matter injury", JOURNAL OF CONTROLLED RELEASE., vol. 214, 1 September 2015 (2015-09-01), pages 112-120, XP55334456, NL ISSN: 0168-3659, DOI: 10.1016/j.jconrel.2015.07.009
- TOLAR J ET AL: "N-acetyl-L-cysteine improves outcome of advanced cerebral adrenoleukodystrophy", BONE MARROW TRANSPLANTATION, NATURE PUBLISHING GROUP, GB, vol. 39, no. 4, 1 February 2007 (2007-02-01), pages 211-215, XP002652784, ISSN: 0268-3369, DOI: 10.1038/SJ.BMT.1705571
- MARC ENGELEN ET AL: "Bezafibrate lowers very long-chain fatty acids in X-linked adrenoleukodystrophy fibroblasts by inhibiting fatty acid elongation", JOURNAL OF INHERITED METABOLIC DISEASE, KLUWER ACADEMIC PUBLISHERS, DO, vol. 35, no. 6, 24 March 2012 (2012-03-24) , pages 1137-1145, XP035123948, ISSN: 1573-2665, DOI: 10.1007/S10545-012-9471-4
- CERQUEIRA SUSANA R ET AL: "Microglia response and in vivo therapeutic potential of methylprednisolone-loaded dendrimer nanoparticles in spinal cord injury.", SMALL (WEINHEIM AN DER BERGSTRASSE, GERMANY) 11 MAR 2013, vol. 9, no. 5, 11 March 2013 (2013-03-11), pages 738-749, XP002768433, ISSN: 1613-6829
- LESNIAK W G ET AL: "Biodistribution of fluorescently labeled PAMAM dendrimers in neonatal rabbits: Effect of neuroinflammation", MOLECULAR PHARMACEUTICS, AMERICAN CHEMICAL SOCIETY, US, vol. 10, no. 12, 2 December 2013 (2013-12-02), pages 4560-4571, XP008178090, ISSN: 1543-8384, DOI: 10.1021/MP400371R [retrieved on 2013-10-11]
- BERGER J ET AL: "Pathophysiology of X-linked adrenoleukodystrophy", BIOCHIMIE, MASSON, PARIS, FR, vol. 98, 4 December 2013 (2013-12-04), pages 135-142, ISSN: 0300-9084, DOI: 10.1016/J.BIOCHI.2013.11.023
- KEMP S ET AL: "GENE REDUNDANCY AND PHARMACOLOGICAL GENE THERAPY: IMPLICATIONS FOR X-LINKED ADRENOLEUKODYSTROPHY", NATURE MEDICINE, NATURE PUBLISHING GROUP US, NEW YORK, vol. 4, no. 11, 1 November 1998 (1998-11-01), pages 1261-1268, ISSN: 1078-8956, DOI: 10.1038/3242
- GONDCAILLE CATHERINE ET AL: "Phenylbutyrate up-regulates the adrenoleukodystrophy-related gene as a nonclassical peroxisome proliferator", THE JOURNAL OF CELL BIOLOGY, vol. 169, no. 1, 11 April 2005 (2005-04-11), pages 93-104, US ISSN: 0021-9525, DOI: 10.1083/jcb.200501036 Retrieved from the Internet: URL:https://www.ncbi.nlm.nih.gov/pmc/artic les/PMC2171887/pdf/200501036.pdf>

## Description

### STATEMENT REGARDING FEDERALLY

### SPONSORED RESEARCH OR DEVELOPMENT

This invention was made with Government Support under Awards 1R01HD076901-01A1 and 1R01HD069562-01 by the National Institutes of Health. The Government has certain rights in the invention.

### FIELD OF THE INVENTION

The field of the invention is generally related to targeted compositions including dendrimer nanodevices for the imaging, diagnosis, and treatment of central nervous system inflammation, particular of the type seen in adrenoleukodystrophy and other leukodystrophies and peroxisomal disorders.

### BACKGROUND OF THE INVENTION

Leukodystrophies are neurodegenerative disorders primarily involving the white matter tracts and are progressive and debilitating. Of these, an extremely severe type is X-linked adrenoleukodystrophy (ALD), which occurs due to mutations in the peroxisomal ABC-transporter, ABCD1, and affects cerebral white matter, spinal cord, and peripheral nerves, with some phenotypes progressing rapidly and terminally at young age (Berger, et al., Biochimie, 98:135-42 (2014)). ALD is biochemically characterized by accumulation of very long chain fatty acids in the nervous system white matter, the adrenal glands and testicles, due to impaired peroxisomal fatty acid metabolism. ABCD1 encodes ALDP, a protein responsible for the import of very long chain fatty acids (VLCFAs) into the peroxisome for degradation, the pathogenic hallmark of ALD, and it is believed that the accumulation of very long chain fatty acid will lead to mitochondrial dysfunction, and oxidative stress. Furthermore, accumulation of very long chain fatty acids in the cell membrane may lead to microglial activation resulting in neuroinflammation.

ALD primarily affects boys who are born normally and have normal initial development. The two most prevalent phenotypes of ALD are the childhood cerebral ALD (ccALD) which is a rapidly progressive, fatal demyelinating cerebral disorder, and the adult onset adrenomyeloneuropathy (AMN), which is a slowly progressive "dying-back" axonopathy of the long tracts in the spinal cord and peripheral nerves (Powers, et al., Journal of neuropathology and experimental neurology, 60(5):493-501 (2001)). Postmortem studies of long tracts in AMN have shown lipidic inclusions in mitochondria suggestive of mitochondrial dysfunction.

About 35% of all males with this genetic defect will present between 4-6 years of age with a rapidly progressive fatal neuroinflammatory demyelinating disorder involving the cerebral white matter. This phenotype defines childhood cerebral ALD (ccALD) and leads to death within 2-3 years after onset of symptoms. The remaining 65% of males will be asymptomatic during childhood but develop an adult onset slowly progressive myelopathy, referred to as adrenomyeloneuropathy (AMN), which is a degenerative long tract axonopathy and progresses over decades and also has a peripheral neuropathy component. Additionally, adult men with AMN carry a 20% chance of developing the same neuroinflammatory demyelinating cerebral disease as in the younger boys and are referred to as adult cerebral ALD (acALD). In addition to the nervous system involvement, nearly all males develop at some point during their lifetime adrenal insufficiency, which can lead to a life threatening emergency, if left untreated. About half of all female carriers will also develop a milder version of AMN but do not develop any neuroinflammation. The total incidence of ALD (males and females combined) is estimated to be 1: 17,000, making ALD the most common leukodystrophy with no ethnic or geographic variation. Newborn screening for this disorder was started on January 1, 2014, in the State of New York, and will likely be expanded to several other high-birth rate states within the next 1-2 years.

The only available therapy for ccALD is allogeneic hematopoietic stem cell transplantation (HSCT), although this procedure is only effective if performed during early disease stages and has a high morbidity and mortality. The mechanism of action is not yet entirely clear, but it is presumed that the exogenous hematopoietic stem cells migrate to the CNS and differentiate into microglia, which arrest the inflammatory demyelination. This implies that targeting microglia may be an effective therapeutic strategy. Several neuromodulatory drugs have been utilized to arrest the inflammatory process (cycophosphamide, IVIG, thalidomide, IFN-δ) without success. A combination of glyceryl trioleate-trierucate, famously referred to as Lorenzo's oil (due to a movie depiction), has been shown to effectively reduce blood very long chain fatty acids, but has not been able to stop disease progression in ccALD. A multicenter trial was initiated of *ex vivo* lentiviral-based gene transduction of autologous hematopoietic stem cells in boys with ccALD who do not have a related bone marrow match and are identified during early disease stages (Study HGB-205: gene therapy for hemoglobinopathies via transplantation of autologous hematopoietic stem cells transduced *ex vivo* with a lentiviral betaa-t87q-globin vector (Lentiglobin BB305 Drug Product, Sponsor: BlueBirdBio, Inc.).

US patent publication no. 2012/003155 was published on 5 January 2012 and was entitled "Dendrimer based nanodevices for therapeutic and imaging purposes".

Manoj K. Mishra et al., "Dendrimer Brain Uptake and Targeted Therapy for Brain Injury in a Large Animal Model of Hypothermic Circulatory Arrest", ACS NANO, US, (20140325), vol. 8, no. 3, pages 2134 - 2147 explored whether systemically administered polyamidoamine (PAMAM) dendrimers could be effective in reaching target cells in the brain and deliver therapeutics in a large animal model.

Elizabeth Nance et al., "Systemic dendrimer-drug treatment of ischemia-induced neonatal white matter injury", Journal of Controlled Release., NL, (20150901), vol. 214, pages 112 - 120 disclose investigations in which a mouse model of ischemia-induced neonatal white matter injury was used to study the biodistribution of generation 4, hydroxyl-functionalized polyamidoamine dendrimers.

International patent publication no. WO 2014/178892 was published on 6 November 2014 and was entitled "Use of sobetirome in the treatment of X-linked adrenolenoleukodystrophy".

International patent publication no. WO 00/18394 was published on 6 April 2000 and was entitled "Adrenoleukodystrophy treatments and drug screening".

In view of the lack of available therapies, there remains a need for improved remedies for treating these disorders.

Therefore, it is an object of the invention to provide compositions and methods of use thereof for treatment of peroxisomal disorders and leukodystrophies.

It is also an object of the invention to provide compositions and methods for target delivery of therapeutic agents to neurons.

It is a further object of the invention to provide compositions and methods for preferential delivery of therapeutic agents to neurons with axonal degeneration over healthy or otherwise undamaged neurons, particularly those located in the spinal cord, more particularly in the gray matter of the spinal cord.

Note that the references to methods of treatment in the summary and detailed description of the invention in this description are to be interpreted as references to the compounds, pharmaceutical compositions and medicaments of the present invention for use in a method for treatment of the human/animal body by therapy or for diagnosis.

### SUMMARY OF THE INVENTION

Compositions, including pharmaceutical compositions and dosage units, and methods of use thereof for diagnosing and treating peroxisomal disorders and leukodystrophies in a subject in need thereof typically include dendrimers complexed, covalently attached or intra-molecularly dispersed or encapsulated with a therapeutic, prophylactic or diagnostic agent for treatment or diagnosis of the disorder.

The invention relates to a dendrimer complex for use in a method of treatment of a peroxisomal disorder in a subject, wherein the complex comprises generation 4-10 poly(amidoamine) (PAMAM) hydroxyl-terminated dendrimers complexed, conjugated or intra-molecularly dispersed or encapsulated with at least one therapeutic or prophylactic agent, wherein the agent is for the treatment of a peroxisomal disorder, and wherein the complex targets microglia.

In some embodiments, the compositions include poly(amidoamine) hydroxyl-terminated dendrimers G4-G10, preferably G4-G6, complexed with therapeutic, prophylactic and/or diagnostic agent in an effective amount to treat, and/or prevent one or more symptoms associated with axonal degeneration. Exemplary therapeutic agents include steroidal anti-inflammatory agents, non-steroidal anti-inflammatory agents, and gold compound anti-inflammatory agents. In some embodiments, the dendrimer is complexed, covalently attached or intra-molecularly dispersed or encapsulated with an anti-inflammatory or antioxidant and an agent such as N-acetylcysteine, 4-phenylbutyrate, bezafibrate, thyroid hormone (T3), sobetirome, pioglitazone, resveratrol, VBP15, Vitamin E, erucic acid, biotin, Coenzyme Q10, clemastine, galactosylceramidase (GALC), or Arylsulfatase A (ARSA). In some embodiments, the therapeutic agents conjugated to the dendrimers are a therapeutically active agent for localizing and targeting Neuron-specific class III beta-tubulin (TUJ-1) positive spinal neurons. In further embodiments, the compositions include the dendrimer with two or more different terminal linkers, and/or spacers, for conjugating with two or more different therapeutic agents.

Methods of administering the compositions are provided to treat one or more symptoms associated with axonal degeneration in individuals with peroxisomal disorders and leukodystrophies, especially an extremely severe type X-linked adrenoleukodystrophy (ALD), which occurs due to mutations in the peroxisomal ABC-transporter, *ABCD1,* and affects cerebral white matter, spinal cord, and peripheral nerves, with some phenotypes progressing rapidly and terminally at young age. The methods typically include systemically administering to the subject an effective amount a pharmaceutically acceptable composition including the dendrimer composition.

The compositions are suitable for use in treatment of peroxisomal disorders that affect the growth or maintenance of the myelin sheath that insulates nerve cells, and leukodystrophies such as adrenoleukodystrophy (ALD) (including X-linked ALD), metachromatic leukodystrophy (MLD), Krabbe disease (globoid leukodystrophy), and Leukoencephalopathy with brainstem and spinal cord involvement and lactate elevation (LBSL)/DARS2 Leukoencephalopathy. In some embodiments, the dendrimers conjugated to therapeutic agent is in a unit dosage in an amount effective to reduce, prevent, or otherwise alleviate oxidative stress, neuroinflammation, long chain fatty acid production, loss of motor function, or a combination thereof; promote, increase, or improve peroxisome proliferation, very long chain fatty acid removal, motor function, ABCD2 expression, enzymes mutated or deficient in peroxisomal disorders or leukodystrophies, or a combination thereof.

### BRIEF DESCRIPTION OF THE DRAWINGS

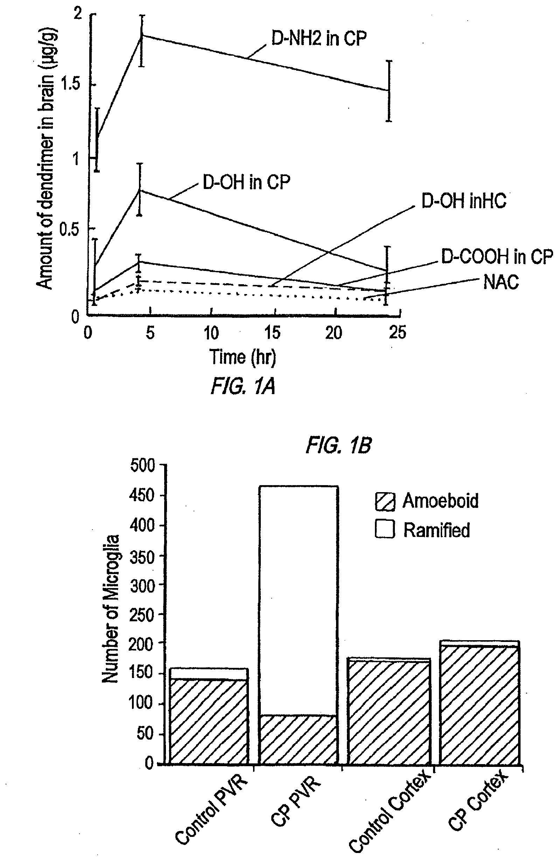

Figure 1A is a graph of the amount of dendrimer in brain (µg/g) over time (hour).
Figure 1B is a graph of the number of microglia versus control, PVR, CP PVR, control cortex and CP cortex.

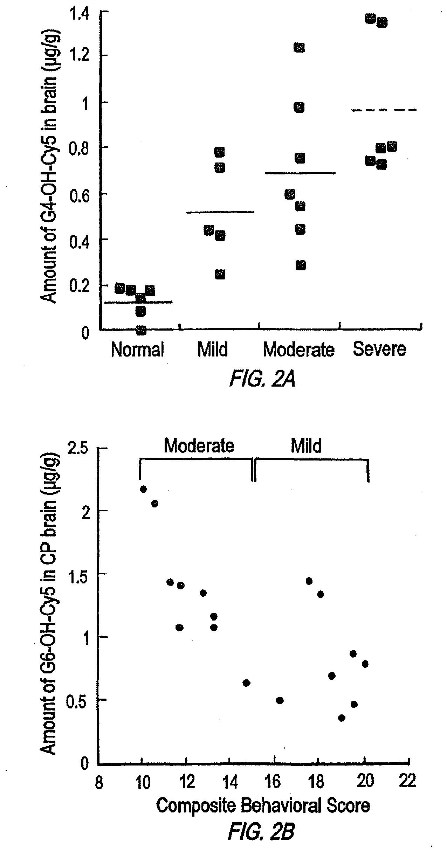

Figure 2A is a graph of amount of G4-OH-Cys5 in brain (µg/ml) for normal, mild, moderate and severe.
Figure 2B is a graph of the amount of G6-OH-Cy5 in CP in brain (µg/ml) composite behavior score.

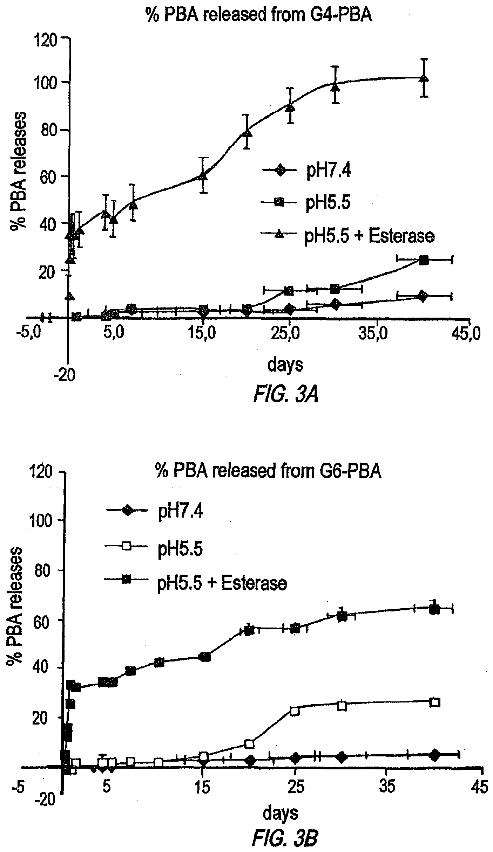

Figures 3A and 3B are plots showing the release of free PBA over a period of 45 days from Dendrimer-PBA conjugates of 4^{th} generation PAMAM dendrimers (FIG.3A), or from 6^{th} generation PAMAM dendrimers (FIG.3B) in pH 7.4 PBS, pH 5.5 citrate buffer, or pH 5.5 in the presence of esterase.

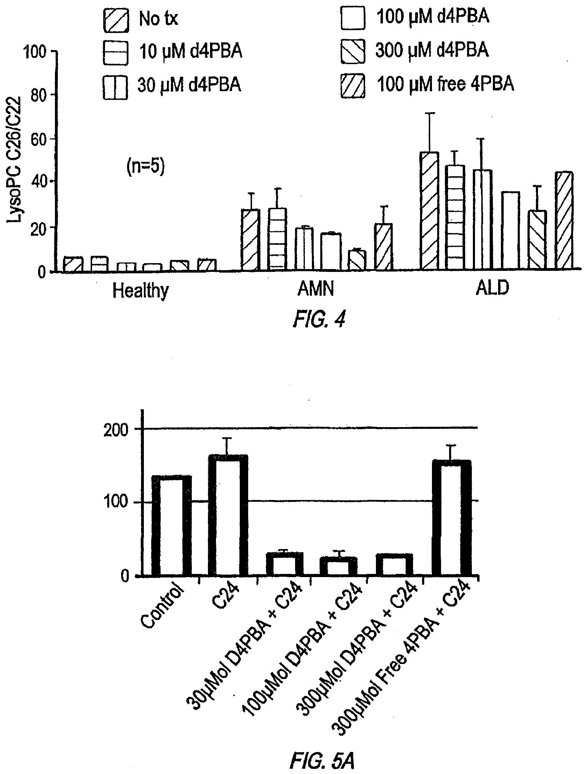

Figure 4 is a bar graph showing LysoPC C26/C22 ratio of fibroblasts derived from healthy patients, or fibroblasts derived from adrenomyeloneuropathy (AMN), or adrenoleukodystrophy (ALD) patients activated by very long chain fatty acid, in the presence of no D4PBA, 10 µM D4PBA, 30 µM D4PBA, 100 µM D4PBA, 300 µM D4PBA, or 100 µM free 4PBA.

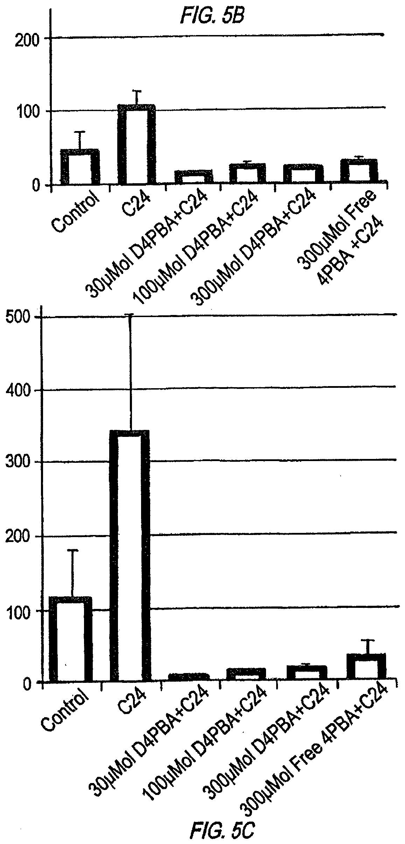

Figures 5A-5C are bar graphs showing TNFα levels in patient-derived mononucleocytes from healthy control (FIG.5A), adrenomyeloneuropathy (AMN) patients (FIG.5B), or adrenoleukodystrophy (ALD) patients (FIG.5C), in the presence or absence of long chain fatty acid (C24), with or without 30 µM D4PBA, 100 µM D4PBA, 300 µM D4PBA, or 300 µM free 4PBA.

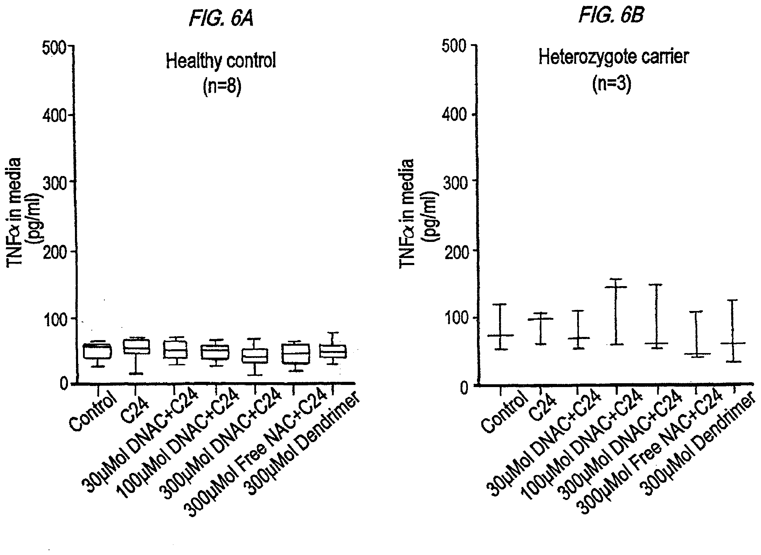

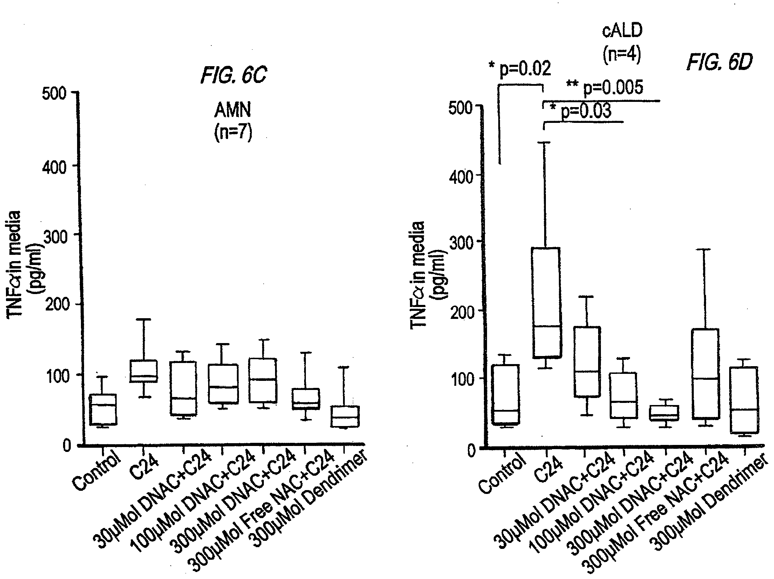

Figures 6A-6D are floating bar charts showing TNFα levels in patient-derived macrophages from healthy control (FIG.6A), heterozyogote carrier (FIG.6B), adrenomyeloneuropathy (AMN) patients (FIG.6C), or cerebral adrenoleukodystrophy (cALD) patients (FIG.6D), in the presence or absence of long chain fatty acid (C24), with or without 30 µM DNAC, 100 µM DNAC, 300 µM DNAC, 300 µM free NAC, or 300 µM Dendrimer.

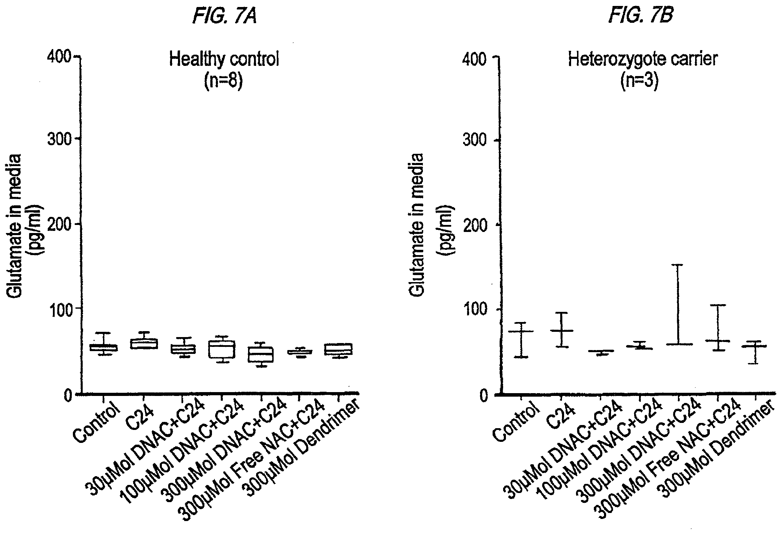

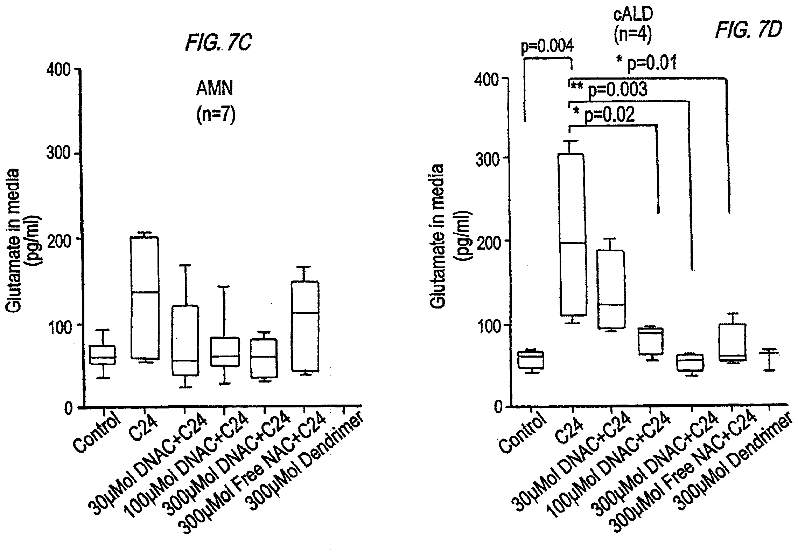

Figures 7A-7D are floating bar charts showing levels of glutamate in patient-derived macrophages from healthy control (FIG.7A), heterozyogote carrier (FIG.7B), adrenomyeloneuropathy (AMN) patients (FIG.7C), or cerebral adrenoleukodystrophy (cALD) patients (FIG.7D), in the presence or absence of long chain fatty acid (C24), with or without 30 µM DNAC, 100 µM DNAC, 300 µM DNAC, 300 µM free NAC, or 300 µM Dendrimer.

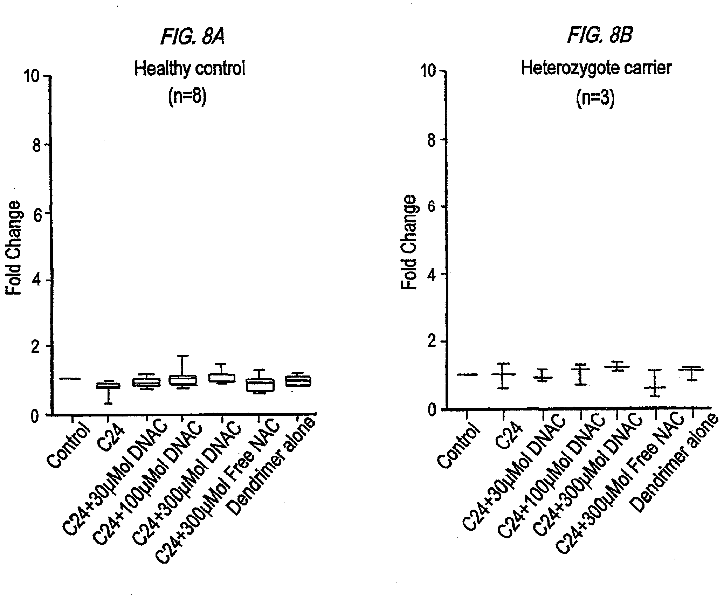

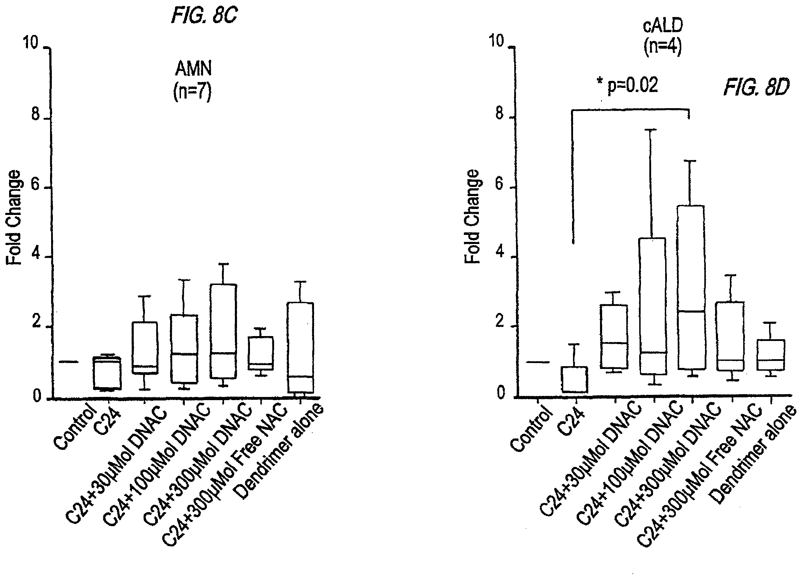

Figures 8A-8D are floating bar charts showing fold changes in glutathione levels in patient-derived macrophages from healthy control (FIG.8A), heterozyogote carrier (FIG.8B), adrenomyeloneuropathy (AMN) patients (FIG.8C), or cerebral adrenoleukodystrophy (cALD) patients (FIG.8D), in the presence or absence of long chain fatty acid (C24), with or without 30 µM DNAC, 100 µM DNAC, 300 µM DNAC, 300 µM free NAC, or 300 µM Dendrimer.

### DETAILED DESCRIPTION OF THE INVENTION

### I. Definitions

The term "therapeutic agent" refers to an agent that can be administered to prevent or treat one or more symptoms of a disease or disorder. Examples include a nucleic acid, a nucleic acid analog, a small molecule, a peptidomimetic, a protein, peptide, carbohydrate or sugar, lipid, or surfactant, or a combination thereof.

The term "treating" refers to preventing or alleviating one or more symptoms of a disease, disorder or condition. Treating the disease or condition includes ameliorating at least one symptom of the particular disease or condition, even if the underlying pathophysiology is not affected, such as treating the pain of a subject by administration of an analgesic agent even though such agent does not treat the cause of the pain.

The term "prevention" or "preventing" means to administer a composition to a subject or a system at risk for or having a predisposition for one or more symptom, caused by a disease or disorder, in an amount effective to cause cessation of a particular symptom of the disease or disorder, a reduction or prevention of one or more symptoms of the disease or disorder, a reduction in the severity of the disease or disorder, the complete ablation of the disease or disorder, stabilization or delay of the development or progression of the disease or disorder.

The term "biocompatible", refers to a material that along with any metabolites or degradation products thereof that are generally non-toxic to the recipient and do not cause any significant adverse effects to the recipient. Generally speaking, biocompatible materials are materials which do not elicit a significant inflammatory or immune response when administered to a patient.

The term "biodegradable", generally refers to a material that will degrade or erode under physiologic conditions to smaller units or chemical species that are capable of being metabolized, eliminated, or excreted by the subject. The degradation time is a function of composition and morphology. Degradation times can be from hours to weeks.

The phrase "pharmaceutically acceptable" refers to compositions, polymers and other materials and/or dosage forms which are, within the scope of sound medical judgment, suitable for use in contact with the tissues of human beings and animals without excessive toxicity, irritation, allergic response, or other problem or complication, commensurate with a reasonable benefit/risk ratio. The phrase "pharmaceutically acceptable carrier" refers to pharmaceutically acceptable materials, compositions or vehicles, such as a liquid or solid filler, diluent, solvent or encapsulating material involved in carrying or transporting any subject composition, from one organ, or portion of the body, to another organ, or portion of the body. Each carrier must be "acceptable" in the sense of being compatible with the other ingredients of a subject composition and not injurious to the patient.

The phrase "therapeutically effective amount" refers to an amount of the therapeutic agent that produces some desired effect at a reasonable benefit/risk ratio applicable to any medical treatment. The effective amount may vary depending on such factors as the disease or condition being treated, the particular targeted constructs being administered, the size of the subject, or the severity of the disease or condition. One of ordinary skill in the art may empirically determine the effective amount of a particular compound without necessitating undue experimentation.

The term "molecular weight", generally refers to the mass or average mass of a material. If a polymer or oligomer, the molecular weight can refer to the relative average chain length or relative chain mass of the bulk polymer. In practice, the molecular weight of polymers and oligomers can be estimated or characterized in various ways including gel permeation chromatography (GPC) or capillary viscometry.

### II. Compositions

### A. Dendrimers

The term "dendrimer" as used herein includes a molecular architecture with an interior core, interior layers (or "generations") of repeating units regularly attached to this initiator core, and an exterior surface of terminal groups attached to the outermost generation. Examples of dendrimers include PAMAM, polyester, polylysine, and PPI. The PAMAM dendrimers have hydroxyl terminations and can be generation 4 PAMAM dendrimers, generation 5 PAMAM dendrimers, generation 6 PAMAM dendrimers, generation 7 PAMAM dendrimers, generation 8 PAMAM dendrimers, generation 9 PAMAM dendrimers, or generation 10 PAMAM dendrimers. Dendrimers used in the method of the invention are polyamidoamine (PAMAM) dendrimers. Other dendrimers suitable for use include polypropylamine (POPAM), polyethylenimine, polylysine, polyester, iptycene, aliphatic poly(ether), and/or aromatic polyether dendrimers. Each dendrimer of the dendrimer complex may be of similar or different chemical nature than the other dendrimers (*e.g.,* the first dendrimer may include a PAMAM dendrimer, while the second dendrimer may comprise a POPAM dendrimer). In some embodiments, the first or second dendrimer may further include an additional agent. The multiarm PEG polymer includes a polyethylene glycol having at least two branches bearing sulfhydryl or thiopyridine terminal groups; however, embodiments disclosed herein are not limited to this class and PEG polymers bearing other terminal groups such as succinimidyl or maleimide terminations can be used. The PEG polymers in the molecular weight 10 kDa to 80 kDa can be used.

A dendrimer complex includes multiple dendrimers. For example, the dendrimer complex can include a third dendrimer; wherein the third-dendrimer is complexed with at least one other dendrimer. Further, a third agent can be complexed with the third dendrimer. In another embodiment, the first and second dendrimers are each complexed to a third dendrimer, wherein the first and second dendrimers are PAMAM dendrimers and the third dendrimer is a POPAM dendrimer. Additional dendrimers can be incorporated. When multiple dendrimers are utilized, multiple agents can also be incorporated. This is not limited by the number of dendrimers complexed to one another.

As used herein, the term "PAMAM dendrimer" means poly(amidoamine) dendrimer, which may contain different cores, with amidoamine building blocks. The method for making them is known to those of skill in the art and generally, involves a two-step iterative reaction sequence that produces concentric shells (generations) of dendritic β-alanine units around a central initiator core. This PAMAM core-shell architecture grows linearly in diameter as a function of added shells (generations). Meanwhile, the surface groups amplify exponentially at each generation according to dendritic-branching mathematics. They are available in generations G0 - 10 with 5 different core types and 10 functional surface groups. The dendrimer-branched polymer may consist of polyamidoamine (PAMAM), polyglycerol, polyester, polyether, polylysine, or polyethylene glycol (PEG), polypeptide dendrimers.

In accordance with the invention, the PAMAM dendrimers used are generation 4-10 dendrimers with hydroxyl groups attached to their functional surface groups. The multiarm PEG polymer comprises polyethylene glycol having 2 and more branches bearing sulfhydryl or thiopyridine terminal groups; however, embodiments are not limited to this class and PEG polymers bearing other terminal groups such as succinimidyl or maleimide terminations can be used. The PEG polymers in the molecular weight 10 kDa to 80 kDa can be used.

In some embodiments, the dendrimers are in nanoparticle form and are described in detail in international patent publication Nos. WO2009/046446, PCT/US2015/028386, PCT/US2015/045112, PCT/US2015/045104, and U.S. Patent No. 8,889,101.

### 1. Preparation of Dendrimer-NAC (D-NAC) - for preparing material suitable for use according to the invention

Below is a synthetic scheme for conjugating *N-*acetylcysteine to an amine-terminated fourth generation PAMAM dendrimer (PAMAM-NH₂), using *N*-succinimidyl 3-(2-pyridyldithio)propionate (SPDP) as a linker. Synthesis of A-succinimidyl 3-(2-pyridyldithio)propionate (SPDP) is performed by a two-step procedure, Scheme 1. First, 3-mercaptopropionic acid is reacted by thiol-disulfide exchange with 2,2'-dipyridyl disulfide to give 2-carboxyethyl 2-pyridyl disulfide. To facilitate linking of amine-terminated dendrimers to SPDP, the succinimide group is reacted with 2-carboxyethyl 2-pyridyl disulfide to obtain *N*-succinimidyl 3-(2-pyridyldithio)propionate, by esterification with N-hydroxysuccinimide by using *N*,*N*'-dicyclohexylcarbodiimide and 4-dimethylaminopyridine.

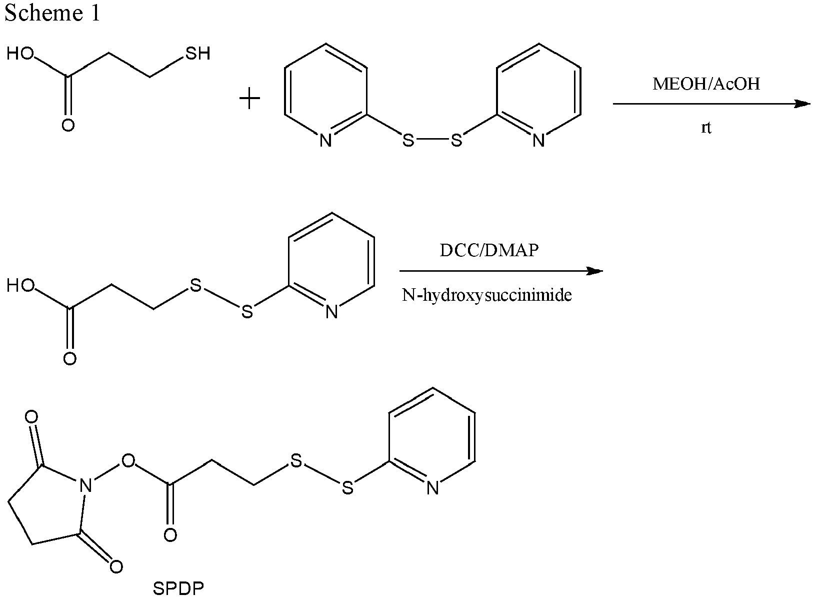

To introduce sulfhydryl-reactive groups, PAMAM-NH₂ dendrimers are reacted with the heterobifunctional cross-linker SPDP, Scheme 2. The N-succinimidyl activated ester of SPDP couples to the terminal primary amines to yield amide-linked 2-pyridyldithiopropanoyl (PDP) groups, Scheme 2. After the reaction with SPDP, PAMAM-NH-PDP can be analyzed using RP-HPLC to determine the extent to which SPDP has reacted with the dendrimers.

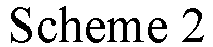

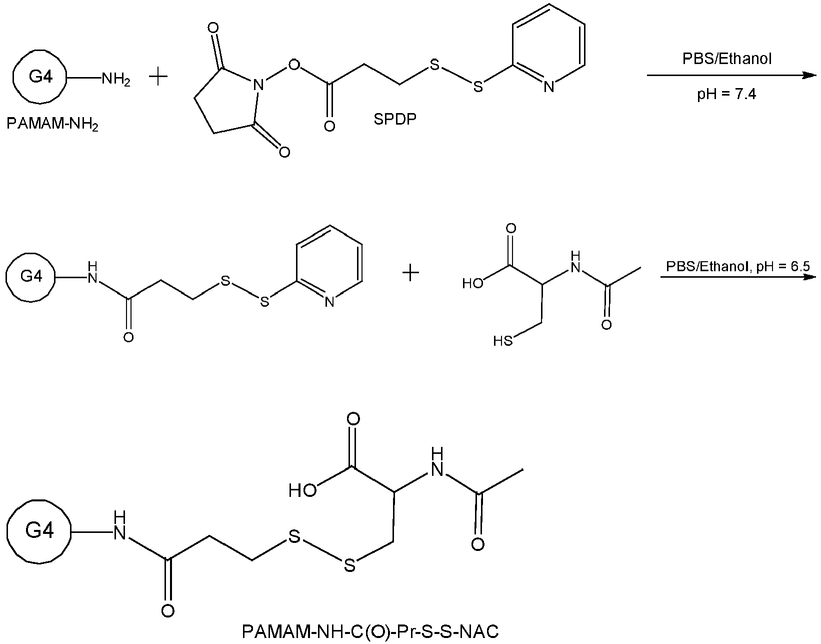

In another embodiment, the synthetic routes described in Scheme 3, below, can be used in order to synthesize D-NAC up to the pyridyldithio (PDP)-functionalized dendrimer (Compound **3).** Compound **3** is then reacted with NAC in DMSO, overnight at room temperature to obtain D-NAC (Compound **5).**

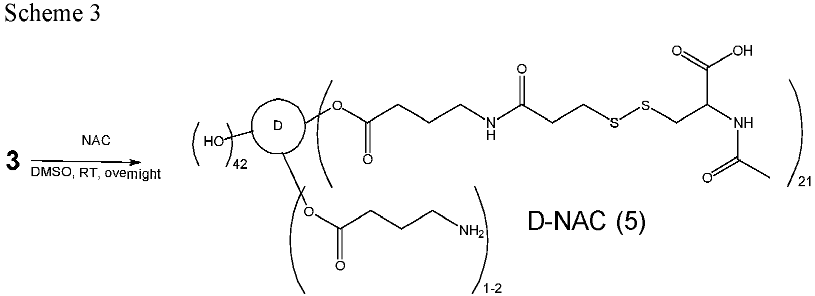

### 2. Preparation of Dendrimer-PEG-valproic acid conjugate (D-VPA)

Initially, valproic acid is functionalized with a thiol-reactive group. A short PEG-SH having three repeating units of (CH₂)₂O- is reacted with valproic acid using DCC as coupling reagent as shown in Scheme 4. The crude PEG-VPA obtained is purified by column chromatography and characterized by proton NMR. In the NMR spectrum, there was a down-shift of the peak of CH₂ protons neighboring to OH group of PEG to 4.25 ppm from 3.65 ppm that confirmed the formation of PEG-VPA. Although the thiol group also may be susceptible to reacting with acid functionality, the NMR spectra did not indicate any downward shift of the peak belonging to CH₂ protons adjacent to thiol group of PEG. This suggests that the thiol group is free to react with the thiol-reactive functionalized dendrimer.

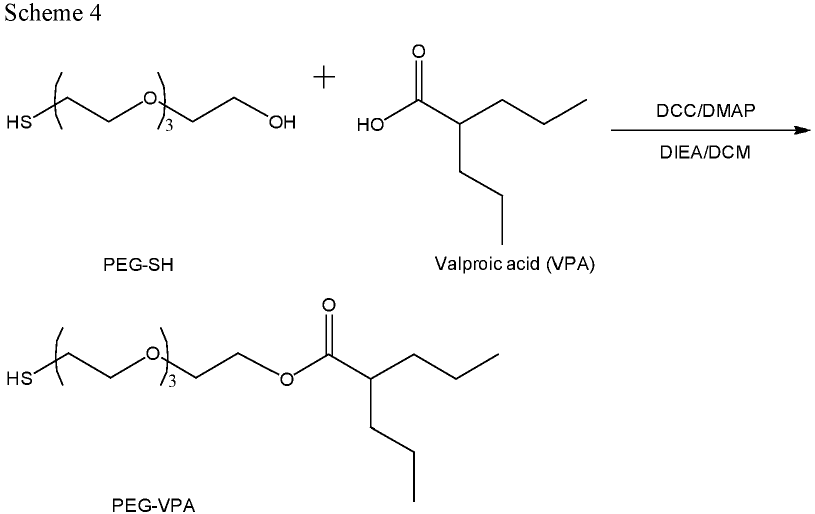

To conjugate PEG-VPA to the PAMAM-OH, a disulfide bond is introduced between the dendrimer and valproic acid, Scheme 5. First the dendrimer is converted to a bifunctional dendrimer (Compound **1)** by reacting the dendrimer with fluorenylmethyloxycarbonyl (Fmoc) protected γ-aminobutyric acid (GABA). Conjugation of PEG-VPA to the bifunctional dendrimer involved a two-step process: the first step is the reaction of amine-functionalized bifunctional dendrimer (Compound **1)** with *N*-succinimidyl-3-(2-pyridyldithio)-propionate (SPDP), and the second step involves conjugating the thiol-functionalized valproic acid. SPDP is reacted with the intermediate (Compound **2** ) in the presence of *N*,*N*-diisopropylethylamine (DIEA) to obtain pyridyldithio (PDP)-functionalized dendrimer (Compound **3).**

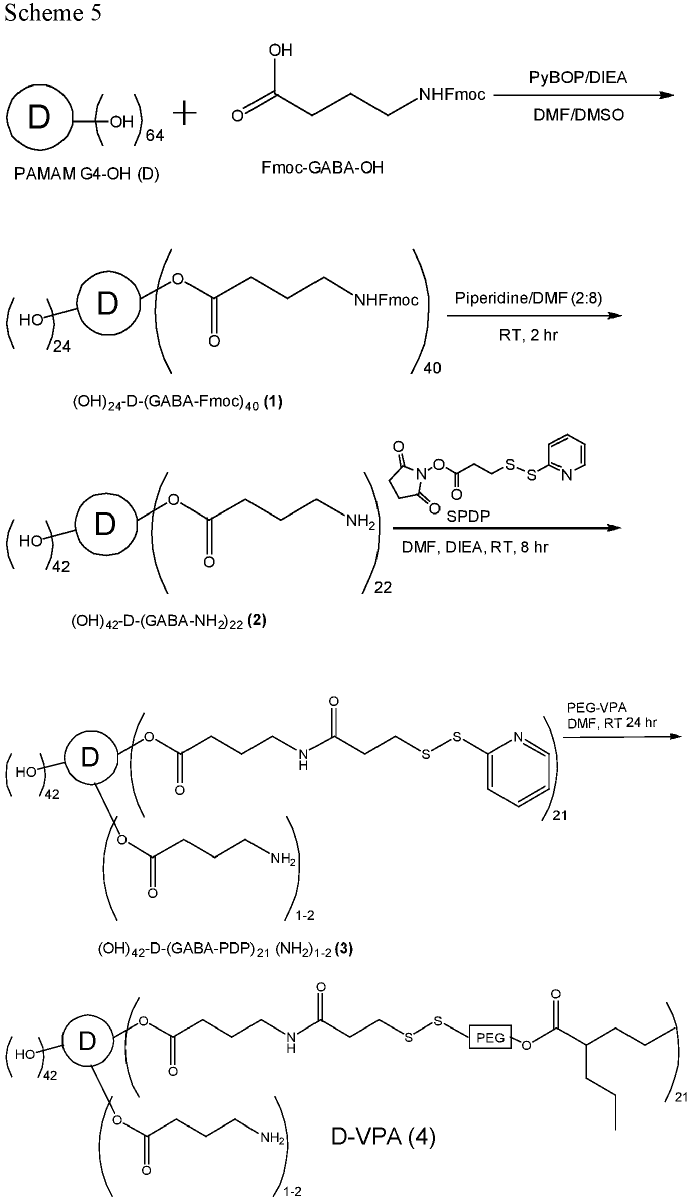

Even though this is an *in situ* reaction process, the structure was established by ¹H NMR. In the spectrum, new peaks between 6.7 and 7.6 ppm for aromatic protons of pyridyl groups confirmed the formation of the product. The number of pyridyl groups and number of GABA linkers were verified to be the same, which indicates that most of the amine groups reacted with the SPDP. Since this is a key step for the conjugation of the drug to the dendrimer, the use of mole equivalents of SPDP per amine group and time required for the reaction was validated. Finally, the PEG-VPA is reacted with the PDP-functionalized dendrimer *in situ* to get dendrimer-PEG-valproic acid (D-VPA). The formation of the final conjugate and loading of VPA were confirmed by ¹H NMR, and the purity of the conjugate was evaluated by reverse-phase HPLC. In the NMR spectrum, multiplets between 0.85 and 1.67 ppm for aliphatic protons of VPA, multiplets between 3.53 and 3.66 ppm for CH₂ protons of PEG, and absence of pyridyl aromatic protons confirmed the conjugate formation. The loading of the VPA is ~21 molecules, estimated using a proton integration method, which suggests that 1-2 amine groups are left unreacted. In the HPLC chart, the elution time of D-VPA (17.2 min) is different from that for G4-OH (9.5 min), confirming that the conjugate is pure, with no measurable traces of VPA (23.4 min) and PEG-VPA (39.2 min).The percentage of VPA loading to the dendrimer is ~12% w/w and validates the method for making gram quantities in three different batches.

### 3. Preparation of Dendrimer-4 phenylbutyrate (D-PBA)

4-phenyl butyric acid (PBA) was conjugated to hydroxyl-functionalized PAMAM dendrimer via a pH labile ester linkage. A propionyl linker was utilized as a spacer both to provide enough space for drug molecules on dendrimer surface and to facilitate their release. Since the attachment of linker is also based on an esterification reaction, a BOC group protection/deprotection strategy was followed to modify PBA molecules and then conjugation to dendrimer surface was performed for both 4th and 6th generation PAMAM dendrimers (Scheme 6).

Since PBA, in its neutralized form, is highly hydrophobic and water insoluble, feed ratio for drug conjugation reactions were kept low in order to obtain a conjugate which is both water soluble and has an enough multivalency with respect to multiple drug molecules attached to the same dendrimer molecule, with the aim of getting improved drug efficacy.

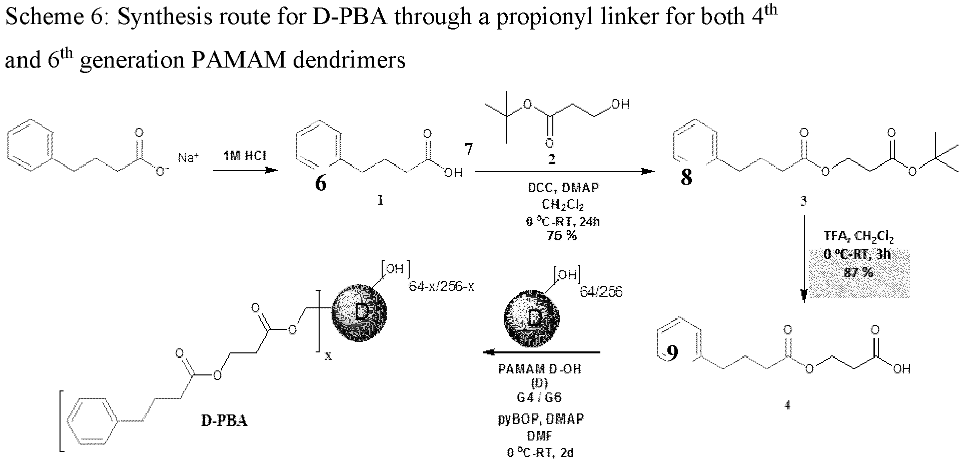

### 4. Preparation of hybrid dendrimer drug conjugates containing two drugs: NAC-Dendrimer-PBA ((G4)-NAC&PBA)

In some embodiments, dendrimers conjugated with two or more different drugs via two or more different linkers are used. As an example, dendrimer conjugate that has two different drugs with two different linkers was successfully synthesized by the attachment of PBA and NAC drug molecules to 4^{th} generation PAMAM dendrimer sequentially. Scheme 7 represents the reaction steps to obtain D-NAC&PBA conjugate.

Based on the nature of functional groups on both drug molecules and linkers, first pyridyl disulfide (PDS) containing propionyl linker was attached to dendrimer via an esterification reaction. Then as a second step, PBA-linker (deprotected) which was already PBA conjugated, was made to react with hydroxyls on dendrimer with the same type of reaction via an ester bond, not to interfere with the carboxylic acid group on NAC molecules afterwards. Lastly, PDS units on the dendrimer were replaced with NAC molecules to form a disulfide bond via disulfide exchange reaction. All the intermediates were purified at each step of the whole synthesis pathway via both dialysis over DMF and precipitation in diethyl ether to give the final conjugate in its pure form.

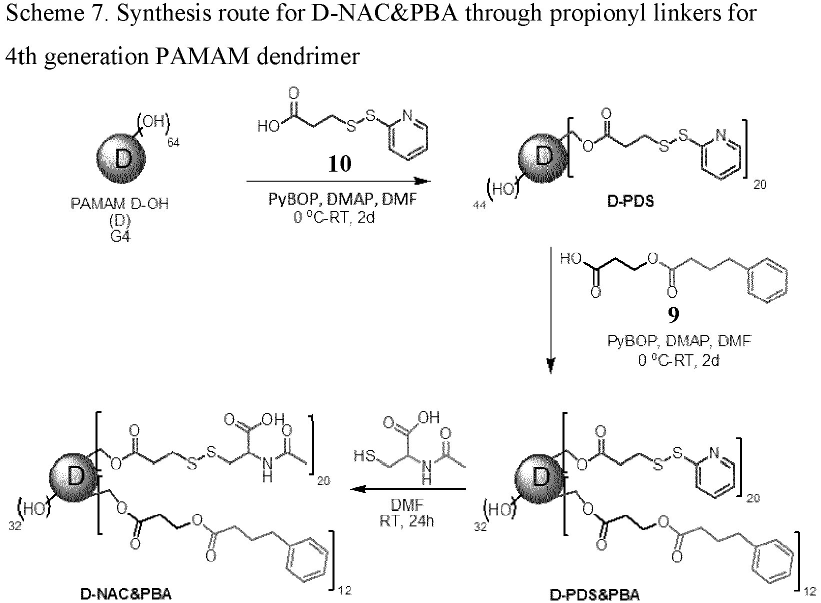

### 5. Preparation of Dendrimer-Bezafibrate (D-BEZA)

Bezafibrate (BEZA) was conjugated to hydroxyl functionalized PAMAM dendrimer via a pH labile ester linkage. The same strategy was applied for the synthesis of bezafibrate-PAMAM conjugates as in the sythesis of D-PBA conjugates. This conjugation depends on the same BOC group protection/deprotection strategy for the sequential esterification reactions, first to attach the linker to bezafibrate, and then to conjugate the drug-linker compound to the dendrimer surface. The same propionyl linker was utilized as a spacer to provide enough space for drug molecules on the dendrimer surface and to facilitate their release. Synthesis of conjugates with bezafibrate was performed for both 4th and 6th generation PAMAM dendrimers (Scheme 8).

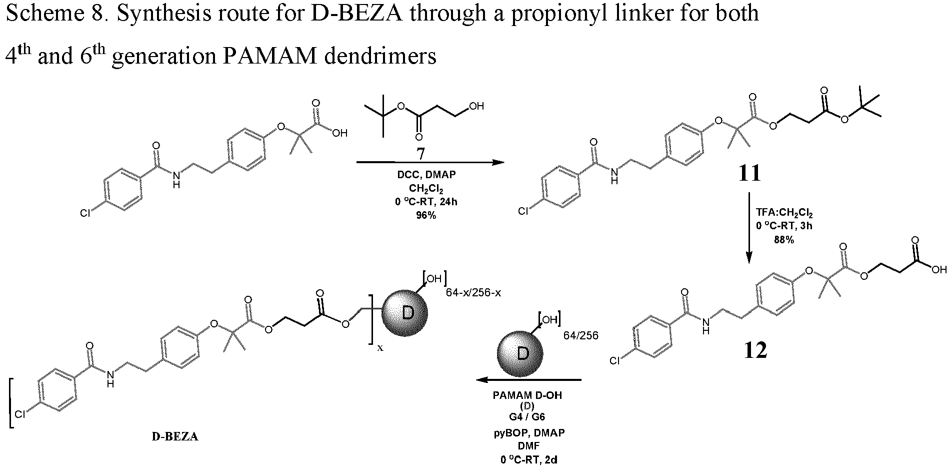

### B. Coupling Agents and Spacers

Dendrimer complexes can be formed of therapeutically active agents or compounds (hereinafter "agent") conjugated or attached to a dendrimer. The attachment can occur via an appropriate spacer that provides a disulfide bridge between the agent and the dendrimer. The dendrimer complexes are capable of rapid release of the agent *in vivo* by thiol exchange reactions, under the reduced conditions found in body.

The term "spacers" as used herein is intended to include compositions used for linking a therapeutically active agent to the dendrimer. The spacer can be either a single chemical entity or two or more chemical entities linked together to bridge the polymer and the therapeutic agent or imaging agent. The spacers can include any small chemical entity, peptide or polymers having sulfhydryl, thiopyridine, succinimidyl, maleimide, vinylsulfone, and carbonate terminations.

The spacer can be chosen from among a class of compounds terminating in sulfhydryl, thiopyridine, succinimidyl, maleimide, vinylsulfone and carbonate group. The spacer can comprise thiopyridine terminated compounds such as dithiodipyridine, N-Succinimidyl 3-(2-pyridyldithio)-propionate (SPDP), Succinimidyl 6-(3-[2-pyridyldithio]-propionamido)hexanoate LC-SPDP or Sulfo-LC-SPDP. The spacer can also include peptides wherein the peptides are linear or cyclic essentially having sulfhydryl groups such as glutathione, homocysteine, cysteine and its derivatives, arg-gly-asp-cys (RGDC), cyclo(Arg-Gly-Asp-d-Phe-Cys) (c(RGDfC)), cyclo(Arg-Gly-Asp-D-Tyr-Cys), cyclo(Arg-Ala-Asp-d-Tyr-Cys). The spacer can be a mercapto acid derivative such as 3 mercapto propionic acid, mercapto acetic acid, 4 mercapto butyric acid, thiolan-2-one, 6 mercaptohexanoic acid, 5 mercapto valeric acid and other mercapto derivatives such as 2 mercaptoethanol and 2 mercaptoethylamine. The spacer can be thiosalicylic acid and its derivatives, (4-succinimidyloxycarbonyl-methyl-α-2-pyridylthio)toluene, (3-[2-pyridithio]propionyl hydrazide, The spacer can have maleimide terminations wherein the spacer comprises polymer or small chemical entity such as bis-maleimido diethylene glycol and bis-maleimido triethylene glycol, Bis-Maleimidoethane, bismaleimidohexane. The spacer can comprise vinylsulfone such as 1,6-Hexane-bis-vinylsulfone. The spacer can comprise thioglycosides such as thioglucose. The spacer can be reduced proteins such as bovine serum albumin and human serum albumin, any thiol terminated compound capable of forming disulfide bonds. The spacer can include polyethylene glycol having maleimide, succinimidyl and thiol terminations.

In some embodiments, two or more different spacers are used on the same dendrimer molecule to conjugate with two or more different drugs.

### C. Therapeutic, Prophylactic and Diagnostic Agents

The term "dendrimer complexes" refers to the combination of a dendrimer with a therapeutically, prophylactically and/or diagnostic active agent. The dendrimers may also include a targeting agent, but as demonstrated by the examples, these are not required for delivery to injured tissue. These dendrimer complexes include one or more agent that is attached or conjugated to PAMAM dendrimers, which are capable of preferentially releasing the drug intracellularly under the reduced conditions found *in vivo.* The dendrimer complex, when administered by i.v. injection, can preferentially localize to damaged or disease neurons, particularly in the gray matter of the spinal cord, over normal cells. The dendrimer complexes are also useful for targeted delivery of the therapeutics in inflammatory disorders, and particularly in peroxisomal diseases and leukodystrophies.

The agent can be either covalently attached or intra-molecularly dispersed or encapsulated. The dendrimer is preferably a PAMAM dendrimer generation 4 to 6, having hydroxyl terminations. The PEG polymer is a star shaped polymer having 2 or more arms and a molecular weight of 10 kDa to 80 kDa. The PEG polymer has sulfhydryl, thiopyridine, succinimidyl, or maleimide terminations. The dendrimer is linked to the agents via a spacer ending in disulfide, ester or amide bonds.

It is believed that in some embodiments, when administered with dendrimer, the dosage of active agent can be lower, the number of administrations can be reduced, or a combination thereof to achieve the same or greater therapeutic effect compared to administering the active agent in the absence of dendrimer. In some embodiments, this allows delivery of agents that are otherwise impractical to administer to a subject in need thereof (1) due to the prohibitively large dose needed to achieve therapeutic effects when the agent is administered absent dendrimer, (2) because the agent when administered alone and untargeted is prohibitively toxic to normal or healthy cells, (3) because active agent is not targeted to the diseased tissue in an effective amount to therapeutically efficacious when alone and untargeted, or (4) a combination thereof.

In some embodiments, two or more active agents are administered to a subject in need thereof. The two or more active agents can be covalently attached or intra-molecularly dispersed or encapsulated in the same or different dendrimers. When two or more dendrimer compositions are utilized, the dendrimers can be of the same or different composition. Furthermore, in some embodiments, one or more active agents are covalently attached or intra-molecularly dispersed or encapsulated in dendrimer, while one or more other active agents are delivered by another suitable means without being covalently attached or intra-molecularly dispersed or encapsulated in dendrimer.

Compositions and formulations including an effective amount of dendrimer and an active agent to treat a peroxisomal disease or leukodystrophy such as ALD are provided. In preferred embodiments, the therapeutic agent is one that reduces, prevents, or otherwise alleviates oxidative stress, neuroinflammation, long chain fatty acid production, loss of motor function, or a combination thereof; promotes, increases, or improves peroxisome proliferation, very long chain fatty acid removal, motor function, ABCD2 expression, expression of wildtype copies of an enzyme mutated or deficient in a peroxisomal disorder or leukodystrophy, or any combination thereof. Preferred active agents include N-acetylcysteine, 4-phenylbutyrate, bezafibrate, thyroid hormone (T3), sobetirome, pioglitazone, resveratrol, VBP15, Vitamin E, galactosylceramidase (GALC), and Arylsulfatase A (ARSA). Other suitable active agents, including anti-inflammatory and imaging agents are also discussed in more detail below.

### 1. Preferred Agents for Treatment of Peroxisomal Diseases and Leukodystrophies

Preferred active agents include agents that prevent or reduce very long chain fatty acid production, agents that promote peroxisome proliferation, promote very long chain fatty acid removal (*e.g.,* 4-phenyl butyrate) agents that increase ABCD2 expression (*e.g.,* benzafibrate), thyromimetics (*e.g.,* sobetirome), enzymes (*e.g.* Galactosylceramidase and Arylsulfatase A, Aspartoacylase), agents that reduce neuroinflammation (e.g, N-acetyl cysteine, Pioglitazone, Vitamin E) and RNA oligonucleotides that interfere with gene transcription or translation. In particularly preferred embodiments, the agent is N-acetylcysteine, 4-phenylbutyrate, bezafibrate, thyroid hormone (T3), sobetirome, pioglitazone, resveratrol, VBP15, Vitamin E, galactosylceramidase (GALC), Aspartoacylase (ASPA), or Arylsulfatase A (ARSA).

### a. N-acetylcysteine

Acetylcysteine, also known as N-acetylcysteine or N-acetyl-L-cysteine (NAC), is a medication used to treat paracetamol (acetaminophen) overdose and diseases include cystic fibrosis and chronic obstructive pulmonary disease. Numerous formulations are known in the art and have been administered numerous routes including intravenous, by mouth, or inhaled as a mist. Numerous commercial formulations are also available and include, for example, ACETADOTE^{®}, which is discussed in U.S. Patent Nos. 8,148,356, 8,399,445, 8,653,061, 8,722,738.

A pilot study of three boys with advanced ccALD who had received N-acetylcysteine (NAC) showed slowing of MRI progression and reversal of gadolinium-contrast enhancement on MRI, a highly predictive marker of disease progression (Tolar, et al., Bone Marrow Transplant, 39(4), 211-215 (2007)). The authors concluded that the anti-oxidative effect of NAC may be beneficial in ccALD. Given that microglial activation and pathology is a key player in ALD and since there is also evidence of oxidative stress and mitochondrial dysfunction, utilization of targeted delivery of NAC to microglia would be an effective way to block disease progression even during later disease stages in ccALD and acALD. Since acALD is a fatal adult disease with no existing therapy, it may be particularly suited for a human trial of dendrimer-N-acetylcysteine. Also, children with advanced stages of ccALD who no longer qualify for HSCT are in great need for a therapeutic intervention.

The boys subject to the study in Tolar, et al., were treated with 140 mg/kg/day intravenously (i.v.) followed by 70 mg/kg four times daily orally of NAC. When administered with dendrimers, the dosage of NAC can be lower, the number of administrations can be reduced, or a combination thereof to achieve the same or greater therapeutic effect compared to administering NAC in the absence of dendrimers.

### b. 4-phenylbutyrate

The active agent can be 4-phenylbutyrate, or 4-phenyl butyric acid. Commercial formulations of sodium phenylbutyrate (4-phenylbutyrate sodium salt) indicated for treatment of urea cycle disorders include BUPHENYL^{®} (sodium phenylbutyrate) (Horizon Pharma), AMMONAPS^{®} (Swedish Orphan Biovitrum International AB), and TRIBUTYRATE^{®} (Fyrlklövern Scandinavia AB). Other formulations include, for example, RAVICTI^{®} (described in U.S. Patent Nos. 5,968,979, 8,404,215, 8,642,012, 9,095,559). In clinical trials the daily dose of sodium phenylbutyrate has been 450 - 600 mg/kg/day in children weighing less than 20 kg, and 9.9 - 13.0 g/m²/day in children weighing more than 20 kg, adolescents and adults.

4-phenylbutyrate treatment of cells from both X-ALD patients and X-ALD knockout mice has been shown to result in decreased levels of and increased beta-oxidation of very-long-chain fatty acids; increased expression of the peroxisomal protein ALDRP; and induction of peroxisome proliferation (Gondcaille, et al., The Journal of cell biology, 169(1):93-104 (2005)). A clinical trial for treatment of ALD has not been pursued due to the need for very high doses in human. When administered with dendrimers, the dosage of 4-phenylbutyrate can be lower, the number of administrations can be reduced, or a combination thereof to achieve the same or greater therapeutic effect compared to administering 4-phenylbutyrate in the absence of dendrimers.

### c. Bezafibrate

The active agent can be bezafibrate. Bezafibrate is a fibrate drug used for the treatment of hyperlipidaemia, and has been investigated for use in treatment of hepatitis C, tauopathy (Dumont, et al., Human Molecular Genetics, 21 (23):5091-5105 (2012), and cancer (University of Birmingham. "Contraceptive, cholesterol-lowering drugs used to treat cancer." ScienceDaily, 14 May 2015; and Southam, et al., Cancer Research, 2015; DOI: 10.1158/0008-5472.CAN-15-0202). Commercial bezafibrate formulations for treatment of hyperlipidaemia include, among others, BEZALIP^{®} (Actavis Group PTC ehf).

Bezafibrate reduces VLCFA levels in X-ALD fibroblasts by inhibiting ELOVL1, an enzyme involved in the VLCFA synthesis (Engelen, et al., Journal of inherited metabolic disease, 35(6): 1137-45 (2012)). However, a clinical trial failed to reduce plasma VLCFA levels in ALD patients while only low plasma levels were achieved (Engelen, et al., PloS one, 7(7):e41013 (2012)). It is believed that targeted delivery to the diseased tissue using dendrimers will increase the therapeutic efficacy of bezafibrate in subjects with ALD, and other leukodystrophies

### d. Thyroid hormone and Thyromimetics

The active agent can be thyroid hormone. In preferred embodiments, the hormone is the thyroid hormone triiodothyronine (T3), or a prohormone thereof. The thyroid hormone triiodothyronine (T3) and its prohormone, thyroxine (T4), are tyrosine-based hormones produced by the thyroid gland that are primarily responsible for regulation of metabolism.

Natural and synthetic T3 and T4, and mixtures thereof, are known in the art and used to treat hypothyroidism. Popular commercial formulations include levothyroxine, a synthetic thyroid hormone that is chemically identical to thyroxine (T4), and liothyronine, a synthetic form of thyroid hormone (T3).

Through its receptor TRβ, T3 can induce hepatic ABCD2 expression in rodents and transiently normalize the VLCFA level in fibroblasts of ABCD1 null mice (Fourcade, et al., Molecular pharmacology, 63(6):1296-303 (2003)). Yet clinical trials with thyroid hormone are unlikely due to the systemic side effects it would exert. Thyroid mimetics are currently under investigation. Administration of thyroid hormone with dendrimers provides an avenue for targeted therapy with reduced systemic toxicity.

In some embodiments, the agent is a thyromimetic. A thyromimetic is an agent that produces effects similar to those of thyroid hormones or the thyroid gland. Exemplary thyromimetics include eprotirome and sobetirome. Thyromimetics that increase the expression of hepatic CYP7A1 include MB07811, KB-141, T-0681, and sobetirome (Pedrelli, et al., World J Gastroenterol., 16(47): 5958-5964 (2010)).

In some embodiments, the active agent is sobetirome. Sobetirome is a thyroid hormone receptor isoform beta-1 liver-selective analog with antilipidemic and antiatherosclerotic activity. In animal models sobetirome reduced serum lipids, decreased cholesterol levels, and stimulated steps of reverse cholesterol transport, which promotes the reverse transport of cholesterol from atherogenic macrophages back to the liver for excretion. In humans, sobetirome lowers plasma LDL cholesterol and reduces plasma triglycerides, while its liver-selective activity helped avoid the side effects seen with many other thyromimetic agents.

### e. Pioglitazone

The active agent can be pioglitazone. Pioglitazone is a thiazolidinedione (TZD) used to treat diabetes. Pioglitazone selectively stimulates the nuclear receptor peroxisome proliferator-activated receptor gamma (PPAR-γ) and to a lesser extent PPAR-α.( Gillies, et al. "Pioglitazone," Drugs, 60(2):333-43 (2000); discussion 344-5. doi: 10.2165/00003495-200060020-00009. PMID 10983737., Smith, et al., J Clin Pract Suppl, (121):13-8 (2001)). Commercial formulations include ACTOS^{®} (Takeda Pharmaceuticals U.S.A., Inc.) which is indicated for glycemic control in adults with type 2 diabetes mellitus in doses of 15 mg, 30 mg, and 45 mg per day.

Pioglitazone has been shown to restore mitochondrial content and expression of master regulators of biogenesis, neutralized oxidative damage to proteins and DNA, and reversed bioenergetic failure in terms of ATP levels, NAD+/NADH ratios, pyruvate kinase and glutathione reductase activities in ABCD1 KO mice (Morato, et al., Brain: a journal of neurology, 136(Pt 8):2432-43 (2013)). Most importantly, the treatment halted locomotor disability and axonal damage in ABCD1 KO mice.

### f. Resveratrol

The active agent can be a resveratrol, such as trans-resveratrol, cis-resveratrol, trans-resveratrol-3-*O*-β-glucoside, or cis-resveratrol-3-*O*-β-glucoside. Resveratrol is a stilbenoid, a type of natural phenol, and a phytoalexin produced by several plants in response to injury or when infected with bacteria or fungi (Fremount, Life Sciences, 66(8):663-673 (2000). Resveratrol has been investigated in anti-aging applications, and to treat heart disease, cancer, Alzheimer's disease, and diabetes. Resveratrol is a Sirt1 inducer, and has also been shown to normalize redox homeostasis, mitochondrial respiration, bioenergetic failure, axonal degeneration and associated locomotor disabilities in the X-ALD mice (Morato, et al., Cell Death and Differentiation, 22:1742-1753 (2015)). In some mouse studies, resveratrol (RSV) (Orchid Chemicals & Pharmaceuticals Ltd, Chennai, India) (0.04% w/w) was mixed into AIN-93G chow from Dyets (Bethlehem, PA, USA) to provide a dose of 400 mg/kg/day (Morato, et al., Cell Death and Differentiation, 22:1742-1753 (2015).

The compound is commercially available for human consumption in the form of nutritional supplements. Some resveratrol capsules sold in the U.S. contain extracts from the Japanese and Chinese knotweed plant *Polygonum cuspidatum* or are made from red wine or red grape extracts. Numerous human doses have been reported ranging from 25 mg to 5,000 mg (Higdon, et al., "Resveratrol," Linus Pauling Institute Micronutrient Information Center, accessed October 2015).

### g. VBP15

The active agent can be VBP15. VBP15 is a steroid analogue, a modified glucocorticoid. Studies in mice showed it is an anti-inflammatory and membrane-stabilizer that improves muscular dystrophy without side effects (Heier, et al, EMBO Mol Med., 5(10): 1569-1585 (2013), Nagaraju, et al., "Delta 9-11 Compound, VBP15: Potential Therapy for DMD" accessed October 2015), and in 2015 it was announced that it would be the subject of a Phase I, first-in-humans clinical trial for treating the same (Olivas, "ReveraGen BioPharma Announces Start of Phase 1 Clinical Trial of VBP15 Dissociative Steroid Drug," media release, February 18, 2015). Dosages in some mouse studies include 5 mg/kg, 15 mg/kg, 30 mg/kg, and 45 mg/kg per day. The compound may also be effective for treating leukodystrophies.

### h. Erucic acid

The active agent can be erucic acid. Erucic Acid is a monounsaturated very long-chain fatty acid with a 22-carbon backbone and a single double bond originating from the 9th position from the methyl end, with the double bond in the cis- configuration. It is prevalent in wallflower seed with a reported content of 20 to 54% in high erucic acid rapeseed oil, and 42% in mustard oil.

When administered with dendrimers, the dosage of erucic acid can be lower, the number of administrations can be reduced, or a combination thereof to achieve the same or greater therapeutic effect compared to administering erucic acid in the absence of dendrimers.

### i. Vitamin E

The active agent can be Vitamin E. Vitamin E refers to a group of compounds that include both tocopherols and tocotrienols. Of the many different forms of vitamin E, γ-tocopherol is the most common form found in the North American diet. γ-Tocopherol can be found in corn oil, soybean oil, margarine, and dressings. α-tocopherol, the most biologically active form of vitamin E, is the second-most common form of vitamin E in the diet. This variant can be found most abundantly in wheat germ oil, sunflower, and safflower oils. As a fat-soluble antioxidant, it interrupts the propagation of reactive oxygen species that spread through biological membranes or through a fat when its lipid content undergoes oxidation by reacting with more-reactive lipid radicals to form more stable products.

When administered with dendrimers, the dosage of Vitamin E can be lower, the number of administrations can be reduced, or a combination thereof to achieve the same or greater therapeutic effect compared to administering Vitamin E in the absence of dendrimers.

### j. Coenzyme Q10

The active agent can be Coenzyme Q10. It is also known as ubiquinone, ubidecarenone, coenzyme Q, and abbreviated at times to CoQ10. It is a 1,4-benzoquinone, where Q refers to the quinone chemical group and 10 refers to the number of isoprenyl chemical subunits in its tail. This fat-soluble substance, which resembles a vitamin, is present in most eukaryotic cells, primarily in the mitochondria. It is a component of the electron transport chain and participates in aerobic cellular respiration, which generates energy in the form of ATP. When administered with dendrimers, the dosage of Coenzyme Q10 can be lower, the number of administrations can be reduced, or a combination thereof to achieve the same or greater therapeutic effect compared to administering Coenzyme Q10 in the absence of dendrimers.

### k. Biotin

The active agent can be biotin. Biotin is a water-soluble B-vitamin, also called vitamin B7, and formerly known as vitamin H or coenzyme R. It is composed of a ureido ring fused with a tetrahydrothiophene ring. A valeric acid substituent is attached to one of the carbon atoms of the tetrahydrothiophene ring. Biotin is a coenzyme for carboxylase enzymes, involved in the synthesis of fatty acids, isoleucine, and valine, and in gluconeogenesis. When administered with dendrimers, the dosage of biotin can be lower, the number of administrations can be reduced, or a combination thereof to achieve the same or greater therapeutic effect compared to administering biotin in the absence of dendrimers.

### 1. Clemastine

The active agent can be clemastine. Clemastine, also known as meclastin, is an antihistamine and anticholinergic. Clemastine fumarate belongs to the benzhydryl ether group of antihistaminic compounds. The chemical name is (+)-2-[-2- [(p-chloro-α-methyl-α-phenylbenzyl) oxy] ethyl]-1-methylpyrrolidine hydrogen fumarate. When administered with dendrimers, the dosage of clemastine can be lower, the number of administrations can be reduced, or a combination thereof to achieve the same or greater therapeutic effect compared to administering clemastine in the absence of dendrimers.

### m. Enzymes

In some embodiments, the active agent is an enzyme, particularly an enzyme whose mutation, deficiency, or other dysregulation is associated with a peroxisomal disease or leukodystrophy. In preferred embodiments, the enzyme is galactosylceramidase (GALC), Aspartoacylase (ASPA), or Arylsulfatase A (ARSA). GALC hydrolyzes galactolipids, including galactosylceramide and psychosine. Galactosylceramide is an important component of myelin. Psychosine forms during the production of myelin, and then it breaks down with help of galactosylceramidase. Krabbe disease is associated with mutations (more than 70 have been identified) in the GALC gene. ARSA is an enzyme that breaks down sulfatides, particularly cerebroside 3-sulfate, into cerebroside and sulfate. Deficiency of ARSA is associated with metachromatic leukodystrophy. Aspartoacylase (ASPA) catalyzes the deacetylation of N-acetylaspartic acid (NAA) to produce acetate and L-aspartate. NAA occurs in high concentration in brain and its hydrolysis NAA plays a significant part in the maintenance of intact white matter. Canavan Disease is associated with mutations in ASPA resulting in accumulation of NAA and spongiform degeneration of cerebral white matter. The agent can be the protein, or a nucleic acid encoding the protein, for example a DNA expression vector or an in vitro transcribed mRNA.

### 2. Other Representative Agents

Other representative therapeutic (including prodrugs), prophylactic or diagnostic agents are also provided. The agents can be peptides, proteins, carbohydrates, nucleotides or oligonucleotides, small molecules, or combinations thereof. The nucleic acid can be an oligonucleotide encoding a protein, for example, a DNA expression cassette or an mRNA.

Exemplary therapeutic agents include anti-inflammatory drugs, antiproliferatives, chemotherapeutics, vasodilators, and anti-infective agents. Antibiotics include β-lactams such as penicillin and ampicillin, cephalosporins such as cefuroxime, cefaclor, cephalexin, cephydroxil, cepfodoxime and proxetil, tetracycline antibiotics such as doxycycline and minocycline, microlide antibiotics such as azithromycin, erythromycin, rapamycin and clarithromycin, fluoroquinolones such as ciprofloxacin, enrofloxacin, ofloxacin, gatifloxacin, levofloxacin and norfloxacin, tobramycin, colistin, or aztreonam as well as antibiotics which are known to possess anti-inflammatory activity, such as erythromycin, azithromycin, or clarithromycin. A preferred anti-inflammatory is an antioxidant drug including N-acetylcysteine. Preferred NSAIDS include mefenamic acid, aspirin, Diflunisal, Salsalate, Ibuprofen, Naproxen, Fenoprofen, Ketoprofen, Deacketoprofen, Flurbiprofen, Oxaprozin, Loxoprofen, Indomethacin, Sulindac, Etodolac, Ketorolac, Diclofenac, Nabumetone, Piroxicam, Meloxicam, Tenoxicam, Droxicam, Lornoxicam, Isoxicam, Meclofenamic acid, Flufenamic acid, Tolfenamic acid, elecoxib, Rofecoxib, Valdecoxib, Parecoxib, Lumiracoxib, Etoricoxib, Firocoxib, Sulphonanilides, Nimesulide, Niflumic acid, and Licofelone.

Representative small molecules include steroids such as methyl prednisone, dexamethasone, non-steroidal anti-inflammatory agents, including COX-2 inhibitors, corticosteroid anti-inflammatory agents, gold compound anti-inflammatory agents, immunosuppressive, anti-inflammatory and anti-angiogenic agents, anti-excitotoxic agents such as valproic acid, D-aminophosphonovalerate, D-aminophosphonoheptanoate, inhibitors of glutamate formation/release, baclofen, NMDA receptor antagonists, salicylate anti-inflammatory agents, ranibizumab, anti-VEGF agents, including aflibercept, and rapamycin. Other anti-inflammatory drugs include nonsteroidal drug such as indomethacin, aspirin, acetaminophen, diclofenac sodium and ibuprofen. The corticosteroids can be fluocinolone acetonide and methylprednisolone. The peptide drug can be streptidokinase.

In some embodiments, the molecules can include antibodies, including, for example, daclizumab, bevacizumab (avastin^{®}), ranibizumab (Lucentis^{®}), basiliximab, ranibizumab, and pegaptanib sodium or peptides like SN50, and antagonists of NF.

Representative oligonucleotides include siRNAs, microRNAs, DNA, and RNA. The therapeutic agent can be a PAMAM dendrimer with hydroxyl terminations.

Exemplary diagnostic agents include paramagnetic molecules, fluorescent compounds, magnetic molecules, and radionuclides, x-ray imaging agents, and contrast media. These may also be ligands or antibodies which are labelled with the foregoing or bind to labelled ligands or antibodies which are detectable by methods known to those skilled in the art.

Exemplary diagnostic agents include dyes, fluorescent dyes, Near infra-red dyes, SPECT imaging agents, PET imaging agents and radioisotopes. Representative dyes include carbocyanine, indocarbocyanine, oxacarbocyanine, thuicarbocyanine and merocyanine, polymethine, coumarine, rhodamine, xanthene, fluorescein, boron-dipyrromethane (BODIPY), Cy5, Cy5.5, Cy7, VivoTag-680, VivoTag-S680, VivoTag-S750, AlexaFluor660, AlexaFluor680, AlexaFluor700, AlexaFluor750, AlexaFluor790, Dy677, Dy676, Dy682, Dy752, Dy780, DyLight547, Dylight647, HiLyte Fluor 647, HiLyte Fluor 680, HiLyte Fluor 750, IRDye 800CW, IRDye 800RS, IRDye 700DX, ADS780WS, ADS830WS, and ADS832WS.

Representative SPECT or PET imaging agents include chelators such as di-ethylene tri-amine penta-acetic acid (DTPA), 1,4,7,10-tetra-azacyclododecane-1,4,7,10-tetraacetic acid (DOTA), di-amine dithiols, activated mercaptoacetyl-glycyl-glycyl-gylcine (MAG3), and hydrazidonicotinamide (HYNIC).

Representative isotopes include Tc-94m, Tc-99m, In-111, Ga-67, Ga-68, Gd³⁺, Y-86, Y-90, Lu-177, Re-186, Re-188, Cu-64, Cu-67, Co-55, Co-57, F-18, Sc-47, Ac-225, Bi-213, Bi-212, Pb-212, Sm-153, Ho-166, and Dy-i66.

Targeting moieties include folic acid, RGD peptides either linear or cyclic, TAT peptides, LHRH and BH3.

The dendrimer complexes linked to a bioactive compound or therapeutically active agent can be used to perform several functions including targeting, localization at a diseased site, releasing the drug, and imaging purposes. The dendrimer complexes can be tagged with or without targeting moieties such that a disulfide bond between the dendrimer and the agent or imaging agent is formed via a spacer or linker molecule.

### D. Devices and Formulations

The dendrimers can be administered parenterally by subdural, intravenous, intrathecal, intraventricular, intraarterial, intra-amniotic, intraperitoneal, or subcutaneous routes.

The carriers or diluents used herein may be solid carriers or diluents for solid formulations, liquid carriers or diluents for liquid formulations, or mixtures thereof.

For liquid formulations, pharmaceutically acceptable carriers may be, for example, aqueous or non-aqueous solutions, suspensions, emulsions or oils. Parenteral vehicles (for subcutaneous, intravenous, intraarterial, or intramuscular injection) include, for example, sodium chloride solution, Ringer's dextrose, dextrose and sodium chloride, lactated Ringer's and fixed oils. Examples of non-aqueous solvents are propylene glycol, polyethylene glycol, and injectable organic esters such as ethyl oleate. Aqueous carriers include, for example, water, alcoholic/aqueous solutions, cyclodextrins, emulsions or suspensions, including saline and buffered media. The dendrimers can also be administered in an emulsion, for example, water in oil. Examples of oils are those of petroleum, animal, vegetable, or synthetic origin, for example, peanut oil, soybean oil, mineral oil, olive oil, sunflower oil, fish-liver oil, sesame oil, cottonseed oil, corn oil, olive, petrolatum, and mineral. Suitable fatty acids for use in parenteral formulations include, for example, oleic acid, stearic acid, and isostearic acid. Ethyl oleate and isopropyl myristate are examples of suitable fatty acid esters.

Formulations suitable for parenteral administration can include antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. Intravenous vehicles can include fluid and nutrient replenishers, electrolyte replenishers such as those based on Ringer's dextrose. In general, water, saline, aqueous dextrose and related sugar solutions, and glycols such as propylene glycols or polyethylene glycol are preferred liquid carriers, particularly for injectable solutions.

Injectable pharmaceutical carriers for injectable compositions are well-known to those of ordinary skill in the art (see, e.g., Pharmaceutics and Pharmacy Practice, J.B. Lippincott Company, Philadelphia, PA, Banker and Chalmers, eds., pages 238-250 (1982), and ASHP Handbook on Injectable Drugs, Trissel, 15th ed., pages 622-630 (2009)).

Formulations for convection enhanced delivery ("CED") include solutions of low molecular weight sales and sugars such as mannitol.

### III. Methods of Use

PCT/US2015/045112 and Kannan, et al., Sci Transl Med., 4(130): 130ra46 (2012) doi: 10. 1 126/scitranslmed. 3 003 162 demonstrate that poly(amidoamine) dendrimers target inflammation in the central nervous system (CNS) and deliver drugs to produce functional improvements in a rabbit model of cerebral palsy. The Examples below show that systemic administration of the dendrimer also leads to significant accumulation of the dendrimer in the injured areas of the spinal cord in mice with ALD, with further selective localization in the inflammatory cells. This selective localization of the dendrimer in the injured brain and spinal cord in these mice has implications for treatment of peroxisomal disorders and leukodystrophies including ALD.

### A. Methods of Treatment

Methods of treating a subject in need thereof are provided. Typically the methods include administering a subject in need thereof with an effective amount of dendrimer complexes including a combination of a dendrimer with one or more a therapeutic or prophylactic and/or diagnostic active agents. The dendrimers may also include a targeting agent, but as demonstrated by the examples, these are not required for delivery to injured tissue in the spinal cord. As discussed above, the dendrimer complexes include an agent that is attached or conjugated to PAMAM dendrimers, which are capable of preferentially releasing the drug intracellularly under the reduced conditions found *in vivo.* The agent can be either covalently attached or intra-molecularly dispersed or encapsulated. The amount of dendrimer complexes administered to the subject can be an effective amount to reduce, prevent, or otherwise alleviate one or more clinical or molecular symptoms of the disease or disorder to be treated compared to a control, for example a subject absent treatment or a subject treated with the active agent alone absent dendrimer. In some embodiments, the amount of dendrimer complexes is effective to reduce, prevent, or otherwise alleviate one or more desired pharmacologic and/or physiologic effects compared to a control, for example a subject absent treatment or a subject treated with the active agent alone absent dendrimer. In particular embodiments, the dendrimer complexes are administered to a subject in need thereof in an effective amount to reduce, prevent, or otherwise alleviate oxidative stress, neuroinflammation, long chain fatty acid production, loss of motor function, or a combination thereof; promote, increase, or improve peroxisome proliferation, long chain fatty acid removal, motor function, ABCD2 expression, expression of wildtype copies of an enzyme mutated or deficient in a peroxisomal disorder or leukodystrophy, or any combination thereof.

In addition those specifically recited above, other suitable physiological and molecular effects and symptoms can be those generally associated with peroxisomal disorders or leukodystrophies or associated with a particular disease or condition, including those discussed in more detail below or otherwise known in the art. In some embodiments, the subject has one or more molecular or clinical symptoms, but has not been diagnosed with a peroxisomal disorder or leukodystrophy, or does not meet the clinical requirements to an affirmative diagnosis. Accordingly, methods of improving each of the disclosed molecular and clinical symptoms disclosed herein in a subject in need thereof by administering the subject an effective amount of dendrimer complexes including an active agent are also each specifically disclosed.

Some of the diseases and disorders discussed in more detail below manifest in infancy or childhood, and can even lead to childhood death. Therefore, in some embodiments, the subject is an infant or child. In some embodiments, the infant is between about birth and about 2 years of age. In some embodiments, the infant is between about birth and about 1 year of age. In some embodiments, the subject is at least one month old (e.g., not a new born). A child can be between about 1 or 2 and about 18 years old. In some embodiments, the child is between about 1 or 2 years of age and about 3, 4, 5, 6, 7, 8, 9, 10, 11, 12, 13, 14, 15, 16, or 17 years of age. Typically, an attending physician will decide the dosage of the composition with which to treat each individual subject, taking into consideration a variety of factors, such as age, body weight, general health, diet, sex, compound to be administered, route of administration, and the severity of the condition being treated. The dose of the compositions can be about 0.0001 to about 1000 mg/kg body weight of the subject being treated, from about 0.01 to about 100 mg/kg body weight, from about 0.1 mg/kg to about 10 mg/kg, and from about 0.5 mg to about 5 mg/kg body weight

In general the timing and frequency of administration will be adjusted to balance the efficacy of a given treatment or diagnostic schedule with the side-effects of the given delivery system. Exemplary dosing frequencies include continuous infusion, single and multiple administrations such as hourly, daily, weekly, monthly or yearly dosing.

Dosing regimens used in the methods can be any length of time sufficient to treat the disclosed diseases and disorders in the subject. The term "chronic" as used herein, means that the length of time of the dosage regimen can be hours, days, weeks, months, or possibly years.

In some embodiments, the dendrimer complexes, with or without a targeting moiety, target neuroinflammatory cells in the brain, neurons in the spinal cord, or a combination thereof. In some embodiments, the dendrimer complexes target Neuron-specific class III beta-tubulin (TUJ-1) positive neurons, particularly those in the spinal cord. In some embodiments, the dendrimer complexes preferentially or selectively target injured, diseased, or disordered neurons compared to non-injured, non-diseased, or non-disordered neurons. As illustrated in the Example below, dendrimers can also accumulate preferentially or selectively in the gray matter compared to the white matter of the spinal cord of the same subject.

### 1. Diseases and Disorders to be Treated

In some embodiments, the peroxisomal disorder is a peroxisome biogenesis disorder. In preferred embodiments the disorder is a peroxisomal disorder or leukodystrophy characterized by detrimental effects on the growth or maintenance of the myelin sheath that insulates nerve cells. The leukodystrophy can be, for example, 18q Syndrome with deficiency of myelin basic protein, Acute Disseminated Encephalomyelitis (ADEM), Acute Disseminated Leukoencephalitis, Acute Hemorrhagic Leukoencephalopathy, X-Linked Adrenoleukodystrophy (ALD), Adrenomyeloneuropathy (AMN), Aicardi-Goutieres Syndrome, Alexander Disease, Adult-onset Autosomal Dominant Leukodystrophy (ADLD), Autosomal Dominant Diffuse Leukoencephalopathy with neuroaxonal spheroids (HDLS), Autosomal Dominant Late-Onset Leukoencephalopathy, Childhood Ataxia with diffuse CNS Hypomyelination (CACH or Vanishing White Matter Disease), Canavan Disease, Cerebral Autosomal Dominant Arteropathy with Subcortical Infarcts and Leukoencephalopathy (CADASIL), Cerebrotendinous Xanthomatosis (CTX), Craniometaphysical Dysplasia with Leukoencephalopathy, Cystic Leukoencephalopathy with RNASET2, Extensive Cerebral White Matter abnormality without clinical symptoms, Familial Adult-Onset Leukodystrophy manifesting as cerebellar ataxia and dementia, Familial Leukodystrophy with adult onset dementia and abnormal glycolipid storage, Globoid Cell Leukodystrophy (Krabbe Disease), Hereditary Adult Onset Leukodystrophy simulating chronic progressive multiple sclerosis, Hypomyelination with Atrophy of the Basal Ganglia and Cerebellum (HABC), Hypomyelination, Hypogonadotropic, Hypogonadism and Hypodontia (4H Syndrome), Lipomembranous Osteodysplasia with Leukodystrophy (Nasu Disease), Metachromatic Leukodystrophy (MLD), Megalencephalic Leukodystrophy with subcortical Cysts (MLC), Neuroaxonal Leukoencephalopathy with axonal spheroids (Hereditary diffuse leukoencephalopathy with spheroids - HDLS), Neonatal Adrenoleukodystrophy (NALD), Oculodetatoldigital Dysplasia with cerebral white matter abnormalities, Orthochromatic Leukodystrophy with pigmented glia, Ovarioleukodystrophy Syndrome, Pelizaeus Merzbacher Disease (X-linked spastic paraplegia), Refsum Disease, Sjogren-Larssen Syndrome, Sudanophilic Leukodystrophy, Van der Knaap Syndrome (Vacuolating Leukodystrophy with Subcortical Cysts or MLC), Vanishing White Matter Disease (VWM) or Childhood ataxia with diffuse central nervous system hypomyelination, (CACH), X-linked Adrenoleukodystrophy (X-ALD), and Zellweger Spectrum disorders including Zellweger Syndrome, Neonatal Adrenoleukodystrophy, Infantile Refsum Disease, Leukoencephalopathy with brainstem and spinal cord involvement and lactate elevation (LBSL), or DARS2 Leukoencephalopathy.

In preferred embodiments, the leukodystrophy is adrenoleukodystrophy (ALD) (including X-linked ALD), metachromatic leukodystrophy (MLD), Krabbe disease (globoid leukodystrophy), or DARS2 Leukoencephalopathy.

The dendrimer compositions typically include generation 4-6 poly(amidoamine) (PAMAM) hydroxyl-terminated dendrimers complexed, covalently attached or intra-molecularly dispersed or encapsulated with at least one therapeutic agent, diagnostic, or imaging agent. In preferred embodiments, the PAMAM dendrimers are generation 6 PAMAM dendrimers. For methods of treatment, the dendrimers can be conjugated to or complexed with therapeutic agent and administered to a subject in an amount effective to alleviate one or more clinical or molecular symptoms of the peroxisomal disorder or leukodystrophy in the subject.

The therapeutic agent can be, for example, one that reduces, prevents, or otherwise alleviates oxidative stress, neuroinflammation, long chain fatty acid production, loss of motor function; promotes, increases, or improves peroxisome proliferation, long chain fatty acid removal, motor function, ABCD2 expression, expression of enzymes mutated or deficient in peroxisomal disorders or leukodystrophies; or any combination thereof.

The therapeutic agent can be an anti-inflammatory or antioxidant. The anti-inflammatory can be a steroidal anti-inflammatory agents, non-steroidal anti-inflammatory agents, or gold compound anti-inflammatory agents. In a particular embodiment the anti-inflammatory is VBP15.

The therapeutic agent can be wildtype copies of an enzyme mutated or deficient in peroxisomal disorders or leukodystrophies or a nucleic acid encoding the enzyme. Exemplary enzymes are galactosylceramidase (GALC) and Arylsulfatase A (ARSA).

The therapeutic agent can be a thyroid hormone or a thyromimetic. In particular embodiments, the thyroid hormone is natural or synthetic triiodothyronine (T3), its prohormone thyroxine (T4), or a mixture thereof. The thyromimetic can be sobetirome.

The therapeutic agent can be an agent that prevents or reduces long chain fatty acid production, promotes peroxisome proliferation, promotes long chain fatty acid removal, or a combination thereof, such as 4-phenyl butyrate. The therapeutic agent can be one that increases ABCD2 expression, such as benzafibrate. The therapeutic agent can reduce neuroinflammation such as N-acetylcysteine, pioglitazone, or vitamin E.

In some embodiments, the therapeutic agent improves redox homeostasis and/or mitochondrial respiration, reduces or reverses bioenergetic failure, axonal degeneration, and/or associated locomotor disabilities, or a combination thereof. An exemplary agent is resveratrol.

The dendrimer complexed, covalently attached or intra-molecularly dispersed or encapsulated with at least two therapeutic agent, for example an anti-inflammatory or antioxidant and an agent selected from the group consisting of N-acetylcysteine, 4-phenylbutyrate, bezafibrate, thyroid hormone (T3), sobetirome, pioglitazone, resveratrol, VBP15, Vitamin E, galactosylceramidase (GALC), and Arylsulfatase A (ARSA). Preferably, the dendrimer complex includes a therapeutically active agent for localizing and targeting Neuron-specific class III beta-tubulin (TUJ-1) positive spinal neurons. The dendrimer conjugates or complexes can be formulated in a suspension, emulsion, or solution.

The dendrimer-therapeutic agent is administered to an individual with a peroxisomal disorder or a leukodystrophy, for example to treat or diagnosis the disorder. The composition can be administered to the subject in a time period selected from the group consisting of: every other day, every three days, every 4 days, weekly, biweekly, monthly, and bimonthly. In some embodiments, the subject is a child, for example, between about birth and 18 years of age. In some embodiments, the subject is between about 1 or 2 year olds and about 1, 2, 3, 4, 5, 6, 7, 8, 9, or 10 years old.

Methods of detecting the presence, location or extent of spinal neuron injury and detecting or diagnosing peroxisomal disorders and leukodystrophies typically include administering a subject in need thereof a dendrimer-diagnostic or imaging agent and then detecting the location of the complex or conjugate in the spinal cord. Methods for monitoring the progression of spinal neuron injury or a symptom of a peroxisomal disorder or leukodystrophy, or monitoring efficacy of a therapeutic agent for treatment of a spinal neuron injury or a symptom of a peroxisomal disorder or leukodystrophy are also disclosed. The methods typically include administering a subject in need thereof a dendrimer-diagnostic agent complex or conjugate and then detecting the location of the complex or conjugate in the spinal cord at a first time point, administering the subject the dendrimer-diagnostic agent complex or conjugate and then detecting the location of the complex or conjugate in the spinal cord at a second time point, and comparing the detection results from the first and second time points to determine if the injury or symptom has worsened, improved, or remained the same.

### a. Peroxisomal Disorders

Peroxisomal disorders are a group of genetically heterogeneous metabolic diseases linked by dysfunction of the peroxisome. Whereas the mitochondria facilitate oxidation of dietary fatty acids (palmitate, oleate and linolate), peroxisomes are responsible for the beta oxidation of very-long-chain fatty acids VLCFAs (C24:0 and C26:0), pristanic acid (from dietary phytanic acid), and dihydroxycholestanoic acid (DHCA) or trihydroxycholestanoic acid (THCA). The two compounds lead to the formation of bile acids, cholic acid, and chenodeoxycholic acid from cholesterol in the liver. Additionally, the peroxisome-based beta-oxidation system enables biosynthesis of polyunsaturated fatty acid (C22:6w3), and assists in the shorting of fatty acid chains, which are in turn degraded in the mitochondria and leading to formation of the acetylcoenzyme A (acetyl-CoA) units utilized in the Krebs cycle to produce energy (adenosine triphosphate [ATP]) (Wanders RJ. "Peroxisomes, lipid metabolism, and human disease." Cell Biochem Biophys. 2000. 32 Spring:89-106.). Peroxisomes also act as intracellular signaling platforms in redox, lipid, inflammatory, and innate immunity signaling (Schonenberger and Kovacs, Front Cell Dev Biol., 3:42, 19 pages (2015), doi: 10.3389/fcell.2015.00042).

In some embodiments, the peroxisome disorder is an isolated enzyme deficiency, a peroxisome degradation disorder, or most preferably a peroxisome biogenesis disorder (PBD). Peroxisome homeostasis is preserved by balancing assembly and biogenesis with degradation of peroxisomes. With respect to peroxisome degradation, three mechanisms have been reported: selective autophagy (pexophagy), proteolysis by peroxisomal Lon protease 2 (LONP2), and 15-lipoxygenase-1 (ALOX15)-mediated autolysis (Till, et al., Int. J. Cell Biol. 2012:512721. 10.1155/2012/512721)). Abnormal accumulation of VLCFAs (C24, C26) is a hallmark of peroxisomal biogenesis disorders. VLCFAs have deleterious effects on membrane structure and function, increasing microviscosity of RBC membranes and damaging the ability of adrenal cells to respond to adrenocorticotropic hormone (ACTH). In the central nervous system, VLCFA accumulation may cause demyelination associated with an inflammatory response in the white matter and increased levels of leukotrienes due to beta-oxidation deficiency (Jedlitschky and Keppler, Adv Enzyme Regul., 33:181-94 (1993)). Associated with this response is a perivascular infiltration by T cells, B cells, and macrophages in a pattern indicative of an autoimmune response. The level of TNF-α is elevated in astrocytes and macrophages at the outermost edge of the demyelinating lesion indicating cytokine-mediated mechanism. VLCFAs are believed to be components of gangliosides and cell-adhesion molecules in growing axons and radial glia, and therefore to contribute to migration defects in the CNS.

Furthermore, biosynthesis of ether phospholipids (including plasmalogen and platelet-activating factor (PAF)) are important for cell membrane integrity, especially in the CNS, and PAF deficiency impairs glutaminergic signaling and has been implicated in human lissencephaly and neuronal migration disorders. Migrational abnormalities are the most likely causes of the severe seizures and psychomotor retardation associated with many types of peroxisomal disorders. The severity of migration defects is correlated with the elevation of VLCFAs, with depressed levels of ether-linked phospholipids, and with elevated levels of bile-acid intermediates (Wanders, et al., Biochim Biophys Acta., 1801(3):272-80 (2010)). Peroxisome biogenesis disorders, and the genetic mutations contributing thereto, are discussed in numerous reviews including, for example, (Powers and Moser, Brain Pathol., 8(1):101-20 (1998); Steinberg, et al., Biochim Biophys Acta., 1763(12): 1733-48 (2006); Khan, et al., J Lipid Res., 51(7): 1685-1695 (2010); Fujiki, et al., Front Physiol., 5:307 (2014), doi: 10.3389/fphys.2014.00307; and Wiesinger, et al., Appl Clin Genet., 8:109-121 (2015)).

Neurological dysfunction is a prominent feature of most peroxisomal disorders (Powers and Moser, Brain Pathol., 8(1):101-20 (1998)). According to Powers, et al., neuropathologic lesions can be divided in three major classes: (i) abnormalities in neuronal migration or differentiation, (ii) defects in the formation or maintenance of central white matter, and (iii) post-developmental neuronal degenerations. Central white matter lesions can be categorized as (i) inflammatory demyelination, (ii) non-inflammatory dysmyelination, and (iii) non-specific reductions in myelin volume or staining with or without reactive astrocytosis. The neuronal degenerations are of two major types: (i) the axonopathy of adrenomyeloneuropathy (AMN) involving ascending and descending tracts of the spinal cord, and (ii) cerebellar atrophy in rhizomelic chondrodysplasia punctata and probably infantile Refsum's disease (IRD).

Prominent peroxisomal disorders include Zellweger syndrome (ZWS), Zellweger-like syndrome, rhizomelic chondrodysplasia punctata type 1 (RCDP1), adrenomyeloneuropathy (AMN), infantile Refsum's disease (IRD), and X-linked adrenoleukodystrophy (X-ALD). Peroxisomal disorders can include a range of symptoms over a range of severity. Common symptoms include facial dysmorphism, CNS malformations, demyelination, neonatal seizures, hypotonia, hepatomegaly, cystic kidneys, short limbs with stippled epiphyses (chondrodysplasia punctata), cataracts, retinopathy, hearing deficit, psychomotor delay, and peripheral neuropathy. Diagnosis is by detecting elevated blood levels of VLCFA, phytanic acid, bile acid intermediates, and pipecolic acid. Experimental treatment with docosahexaenoic acid (DHA-levels of which are reduced in patients with disorders of peroxisome formation) has shown some promise (Fong, "Peroxisomal Disorders," Merck Manuals Profession Edition (2010)).

### b. Leukodystrophies

In some embodiments, the disorder is a leukodystrophy. Peroxisomal disorders that include effects on the growth or maintenance of the myelin sheath that insulates nerve cells are referred to as leukodystrophies. Leukodystrophies are rare, typically progressive, genetic disorders.

The United Leukodystrophy Foundation reports that up to forty leukodystrophies have been identified, including 18q Syndrome with deficiency of myelin basic protein, Acute Disseminated Encephalomyelitis (ADEM), Acute Disseminated Leukoencephalitis, Acute Hemorrhagic Leukoencephalopathy, X-Linked Adrenoleukodystrophy (ALD), Adrenomyeloneuropathy (AMN), Aicardi-Goutieres Syndrome, Alexander Disease, Adult-onset Autosomal Dominant Leukodystrophy (ADLD), Autosomal Dominant Diffuse Leukoencephalopathy with neuroaxonal spheroids (HDLS), Autosomal Dominant Late-Onset Leukoencephalopathy, Childhood Ataxia with diffuse CNS Hypomyelination (CACH or Vanishing White Matter Disease), Canavan Disease, Cerebral Autosomal Dominant Arteropathy with Subcortical Infarcts and Leukoencephalopathy (CADASIL), Cerebrotendinous Xanthomatosis (CTX), Craniometaphysical Dysplasia with Leukoencephalopathy, Cystic Leukoencephalopathy with RNASET2, Extensive Cerebral White Matter abnormality without clinical symptoms, Familial Adult-Onset Leukodystrophy manifesting as cerebellar ataxia and dementia, Familial Leukodystrophy with adult onset dementia and abnormal glycolipid storage, Globoid Cell Leukodystrophy (Krabbe Disease), Hereditary Adult Onset Leukodystrophy simulating chronic progressive multiple sclerosis, Hypomyelination with Atrophy of the Basal Ganglia and Cerebellum (HABC), Hypomyelination, Hypogonadotropic, Hypogonadism and Hypodontia (4H Syndrome), Lipomembranous Osteodysplasia with Leukodystrophy (Nasu Disease), Metachromatic Leukodystrophy (MLD), Megalencephalic Leukodystrophy with subcortical Cysts (MLC), Neuroaxonal Leukoencephalopathy with axonal spheroids (Hereditary diffuse leukoencephalopathy with spheroids - HDLS), Neonatal Adrenoleukodystrophy (NALD), Oculodetatoldigital Dysplasia with cerebral white matter abnormalities, Orthochromatic Leukodystrophy with pigmented glia, Ovarioleukodystrophy Syndrome, Pelizaeus Merzbacher Disease (X-linked spastic paraplegia), Refsum Disease, Sjogren-Larssen Syndrome, Sudanophilic Leukodystrophy, Van der Knaap Syndrome (Vacuolating Leukodystrophy with Subcortical Cysts or MLC), Vanishing White Matter Disease (VWM) or Childhood ataxia with diffuse central nervous system hypomyelination, (CACH), X-linked Adrenoleukodystrophy (X-ALD), and Zellweger Spectrum disorders including Zellweger Syndrome, Neonatal Adrenoleukodystrophy, and Infantile Refsum Disease.

In particular embodiments, the disorder is adrenoleukodystrophy (ALD) (including X-linked ALD), metachromatic leukodystrophy (MLD), or Krabbe disease (globoid leukodystrophy). The disorder can be a hereditary leukoencephalopathy with brainstem and spinal cord involvement (lesions) and leg spasticity such as DARS2 Leukoencephalopathy, which is caused by mutations in the mitochondrial aspartyl tRNA-synthetase encoding gene (Wolf, et al., Neurology, 84(3):226-30 (2015)).

In a particularly preferred embodiment, the disorder is X-linked adrenoleukodystrophy (X-linked ALD), a monogenic disease caused by mutations in the ABCD1 gene located on Xq28.1 (reviewed in Wiesinger, et al., Appl Clin Genet., 8:109-121 (2015)). The ABCD1 gene codes for the peroxisomal transporter ATP-binding cassette subfamily D member 1 (ABCD1, formerly ALDP), which mediates the import of very long-chain fatty acid (VLCFA) CoA esters across the peroxisomal membrane.

Clinically, X-ALD can present with a wide range of phenotypes (Engelen, et al., Orphanet J Rare Dis. 2012;7:51, and Moser et al., In: Scriver R, et al. editors. The Metabolic and Molecular Bases of Inherited Disease. 8th ed. New York, NY, USA: McGraw-Hill Book Co; 2001.). Two major phenotypes are adrenomyeloneuropathy (AMN) and the cerebral form of X-ALD (CALD). Sixty-five percent of X-linked ALD in males present as AMN, which is characterized by slowly progressive axonopathy. The first symptoms in males usually appear between 20 and 30 years of age, while affected females may develop some symptoms of AMN with an average onset between 40 and 50 years. Twenty percent of these subjects will develop the cerebral form and rapidly progress adult cerebral ALD (acALD). Symptoms of acALD are similar to those of schizophrenia and can include, for example, dementia. The progression of the disorder is rapid, with the average time from the initial symptoms to vegetative state or death being approximately 3-4 years.

CALD usually only affects males and presents with rapidly progressive inflammatory demyelination in the brain, leading to rapid cognitive and neurological decline (Moser et al., In: Scriver R, et al. editors. The Metabolic and Molecular Bases of Inherited Disease. 8th ed. New York, NY, USA: McGraw-Hill Book Co; 2001; Semmler, et al., Expert Rev. Neurother, 8:1367-1379 (2008)). The mutation in ABCD1 is needed, but not sufficient, for CALD to occur, because additional genetic or environmental factors are required to trigger the brain inflammation. Thirty-five percent of X-linked ALD in males present at 4-6 years of age as childhood cerebral ALD, which is typically fatal within 2-3 years after diagnosis.

Almost all adult males with ALD, as well as some female carriers, develop adrenal insufficiency. ALD is a rare disorder with an over frequency (Males+Females) of 1:17,000. The dysfunction of ABCD1 results in impaired degradation of VLCFAs in peroxisomes leading to their accumulation in various lipid species in tissues and body fluids (Di Biase et al., Neurochem. Int. 44:215-221 (2004)). While accumulation of VLCFAs is believed to directly contribute to the demyelinating pathology in AMN, the molecular mechanism by which VLCFAs are involved in the onset or progression of inflammation in CALD is still not entirely clear. Methods of diagnosis include analysis of biomarkers including VLCFAs accumulated in plasma, leucocytes, and fibroblasts from X-ALD patients, which can occur independent of phenotype. Thus, an elevated level of VLCFAs represents the standard biomarker for diagnosis of X-ALD, but does not predict the phenotype or progression of disease. Other diagnostic markers include microglial activation, blood-brain-barrier impairment, and neuroinflammation (Eichler, et al., Ann Neurol., 63(6):729-42 (2008) doi: 10. 1002/ana.21391).

In some particular embodiments, subjects with an ALD, such as ccALD or caALD are administered an effective amount of dendrimer complexes including *N*-acetylcysteine (NAC). Oxidative stress is a major mechanism of injury underlying axonal degeneration (Galea, et al., Biochim Biophys Acta., 1822(9):1475-88 (2012) doi: 10.1016/j.bbadis.2012.02.005), and it is believed that dendrimer-NAC complexes can overcome impaired blood-brain-barrier and target the microglia while serving as both an antioxidant and/or an anti-inflammatory to reduce one or more molecular symptoms, one or more clinical symptoms, or preferably a combination thereof.

In some embodiments, the subjects are between about 2 and 17 years of age, have a MRI LOES score (Loes, et al., AJNR Am J Neuroradiol, 15:1761-1766 (1994)) of between about 9 and 16, exhibit a progression of loss of cognitive function and/or increased neurological symptoms, or a combination thereof.

In some embodiments, the dendrimer complexes are administered to a subject in need thereof in an effective amount to reduce or inhibit peroxisomal beta oxidation, glutamate secretion, one or more pro-inflammatory cytokines, or any combination thereof, in one or more cell types involved in the pathogenesis of a peroxisomal disorder, leukodystrophy, or any combination thereof.

In some embodiments, the dendrimer complexes are administered to a subject in need thereof in an effective amount to reduce or inhibit protein expression and/or secretion of one or more pro-inflammatory cytokines in one or more cell types involved in the pathogenesis of a peroxisomal disorder, leukodystrophy, or any combination thereof. Exemplary pro-inflammatory cytokines include IL1α, IL1β, IL2, IL6, IL8, and TNFα. Typically, the compositions are effective in reducing the activity and/or quantity of one or more pro-inflammatory cytokines in one or more cell types, for example, in microglia/macrophage. In some embodiments, the compositions lead to direct, and/or indirect reduction of one or more pro-inflammatory cytokines such as TNFα by 5%, 10%, 20%, 30%, 40%, 50%, 60%, 70%, 80%, 90%, or more than 90%. In some embodiments, the compositions lead to direct, and/or indirect reduction in glutamate secretion and/or expression by 5%, 10%, 20%, 30%, 40%, 50%, 60%, 70%, 80%, 90%, or more than 90%.

In some embodiments, the dendrimer complexes are administered to a subject in need thereof in an effective amount to increase glutathione expression in one or more cell types involved in the pathogenesis of a peroxisomal disorder, leukodystrophy, or any combination thereof. In some embodiments, the compositions lead to direct, and/or indirect increase in glutathione levels by 10%, 20%, 30%, 40%, 50%, 60%, 70%, 80%, 90%, 100%, 200%, 300%, 400% or more than 400%.

### 2. Combination Therapies

The dendrimer complexes can be administered in combination with one or more additional therapeutically active agents, particularly those which are known to be capable of treating conditions or diseases discussed above, and/or with other remedies such as bone-marrow transplantation. Other exemplary combinations includes co-treatment with symptomatic therapy of adrenal or gonadal insufficiency, neuropathic pain, and spasticity (Singh, Methods Enzymol, 352:361-372 (2002)).

The combination therapies can include administration of the active agents, dendrimer complexes, or combinations thereof together in the same admixture, or in separate admixtures. Therefore, in some embodiments, the pharmaceutical composition includes two, three, or more active agents. The different active agents can have the same mechanism or different mechanisms of action. In some embodiments, the combination results in an additive effect on the treatment of the disease or disorder. In some embodiments, the combinations results in a more than additive effect on the treatment of the disease or disorder. The pharmaceutical compositions can be formulated as a pharmaceutical dosage unit, also referred to as a unit dosage form.

In some embodiments, dendrimer complexes are administered as an adjunct to bone marrow transplantation, particularly in a subject with ALD or another dystrophy. It is generally recognized that oxidative stress and inflammation are detrimental to stem cell survival and growth. Therapy with dendrimer complexes can treat inflammation and oxidative stress in the brain and promote stem cell survival. Bone marrow transplantation is a particularly viable treatment when brain inflammation is detected early (Fourcade, et al., Hum. Mol. Genet. 17: 1762-1773 (2008)). However, hematopoietic stem cell therapy (HSCT) is believed to only arrest the inflammatory demyelination and not impact the non-inflammatory axonopathy (Wheeler, et al., Brain, 131: 3092-3102 (2008), therefore, by itself it is generally not considered to be a therapeutic option for AMN patients without inflammatory involvement.

### B. Diagnostic Methods

The selective localization of dendrimer tagged with an imaging agent to inflammatory cells can also be used a diagnostic tool for early detection of neuroinflammation in susceptible patients. In some embodiments, the dendrimer tagged with an imaging agent, with or without a targeting moiety, can target neuroinflammatory cells in the brain, neurons in the spinal cord, or a combination thereof. In some embodiments, the dendrimer-based imaging agents target TUJ-1 positive neurons, particularly those in the spinal cord. In some embodiments, the dendrimer-based imaging agents preferentially or selectively target injured, diseased, or disordered neurons compared to non-injured, non-diseased, or non-disordered neurons.

Suitable imaging agents are discussed in more detail above and methods of detecting neuroinflammation using imaging and contrast agents are well known in the art. For example, in some embodiments, a subject in need thereof is administered an effective amount of dendrimer complexes including an imaging agent to localize to the target cells or tissue. The subject can be scanned or imaged to detect the dendrimer complexes. Imaging procedures include X-ray radiography, magnetic resonance imaging, medical ultrasonography or ultrasound, endoscopy, elastography, tactile imaging, thermography, medical photography and nuclear medicine functional imaging techniques as positron emission tomography. The imaging or contrast agent can be selected based on the desired imaging or scanning technique utilized, or vice versa.

In some embodiments, a series of scans or images are taken at different time points (e.g., hours, days, weeks, months, or years apart) and compared to monitor the progression of a disease or disorder over a period of time. In some embodiments, the subject is administered a treatment for the disease or disorder over the period of time and the scans or images are compared to review, analyze, or otherwise determine the affect or efficacy of the treatment. Treatments include those disclosed here as well as other that are conventional or otherwise known in the art for treatment the disease or disorder. Disease and disorders include neuorinflammation and injury in the brain and/or spinal cord, as well as the peroxisomal disorders and leukodystrophies such as those discussed above.

In some embodiments, the subject is imaged by MRI and evaluated using the LOES scale (see, *e.g.,* Loes, et al., AJNR Am J Neuroradiol, 15:1761-1766 (1994)). The detection methods utilizing dendrimer complexes can be employed for non-invasive, real-time detection of CNS inflammation for early detection and diagnosis, and treatment monitoring of ALD and other peroxisome disorders and leukodystrophies before symptoms develop and before they can be detected by standard MRI techniques.

### IV. Kits

Medical kits including containers holding one or more of the compositions including dendrimers, dendrimer complexes, or other disclosed agents, are also provided. The kits can optionally include pharmaceutical carriers for dilution thereof and instructions for administration. In addition, two or more of the compositions can be present as components in a single container, in a pharmaceutically acceptable carrier, for co-administration. The compositions or pharmaceutical compositions thereof can also be provided in dosage units.

### Example

### Example 1: Treatment of ALD in Mouse Model

### Materials and Methods

### Mouse model of Adrenoleukodystrophy (ALD)

Adenoleukodystrophy (ALD) is an X-linked disease affecting cerebral white matter and spinal cord, some phenotypes progressing rapidly and terminally at young age. A common mouse model used is the ABCD1 knockout mouse. ABCD1 encodes ALDP, a protein responsible for the import of very long chain fatty acids (VLCFAs) into the peroxisome for degradation, the pathogenic hallmark of ALD. In the mouse model, this leads to increased serum VLCFAs, higher markers of oxidative stress and has shown axonal damage in the spinal cord at 3.5 months (Galino, et al., Antioxidants & redox signaling, 15(8):2095-2107 (2011)). Aging ABCD1 KO mice also exhibit an abnormal neurological and behavioral phenotype, starting at around 15 months (Pujol, et al., Human molecular genetics, 11(5):499-505 (2002)). This is correlated with slower nerve conduction, and axonal anomalies detectable in the spinal cord and sciatic nerve as seen in electron microscopy, resembling the human AMN phenotype. Several antioxidants have been shown to halt axonal degeneration in the ABCD1 KO mouse, yet it is difficult to deliver equivalent therapeutic doses to patients with ALD (Lopez-Erauskin, et al., Annals of neurology, 70(1):84-92 (2011)).

### Dendrimer Administration

6 Month old ABCD1 KO mice were injected with Cy5-labeled dendrimer (D-Cy5) through intraperitoneal administration at a dose of 20 mg/kg, and euthanized at 24 hours post D-Cy5 administration, followed by whole animal perfusion fixation. Perfusion is performed using first phosphate buffered saline (PBS), then 4% Paraformaldehyde solution into the circulatory system. Whole spine removal is performed by removing the dorsal skin and paravertebral muscles, laminectomy of the vertebral pedicles and disconnection to spinal ganglia along the entire length of the spinal cord.

### Immunohistochemistry study

To further process the collected spine for immunohistochemistry study, rodent spine is fixated at 4°C in 4% formalin solution for 24hr, following with processing with sucrose gradient. Spine is frozen in Optimal Cutting Temperature (OCT) solution and cryosectioned into cervical (~10 slices), thoracic (~10 slices) and lumbar (~5 slices) sections, with each slice have a thickness of 10-15 µm. To study the D-Cy5 distribution and neuronal uptake localization, mouse spinal cord slices were stained with anti-beta III tubulin antibody (TUJ-1, labelled with Alexa Fluor^{®} 488) (Abcam, USA), to study the D-Cy5 localization in microglia/macrophage, mouse spinal cord slices were stained with rabbit anti-Ibal antibody (Wako, Japan), following with donkey anti rabbit Alexa flour 488 secondary antibody (Lifetechnology, USA). 4',6-diamidino-2-phenylindole (DAPI) was used to stain cell nuclei in all slices. For confocal study, each image was taken under the same imaging settings.

### Results

D-Cy5 accumulation in the ALD and wild type (WT) mice in cervical, thoracic and lumbar sections were imaged. D-Cy5 had significantly higher accumulation in the spinal cord of ALD mice than wild type. For the spinal cord section of ALD mice, gray matter showed higher D-Cy5 accumulation relative to white matter. Images were taken under 10X with tile scan using confocal microscope. DAPI was utilized to visualize nuclei.

Higher magnification showed D-Cy5 was mostly taken up by the neuron (staining by TUJ 1) in the gray matter spinal cord sections of the ALD mice. The analysis also showed that in the WT mice, there were some D-Cy5 taken up by neurons (staining by TUJ 1) in the gray matter of spinal cord, but was not significant compared with ALD mice. Images were taken under 40X with tile scan using confocal microscope. DAPI was utilized to visualize nuclei. TUJ 1 detection was utilized to identify neuron cells.

In summary, these results show:
1. Dendrimers were found to mostly accumulate at the gray matter of spinal cord in the ALD mice.
2. Dendrimers were mostly taken up by the neurons in the spinal cord of ALD mice. The localization of the dendrimers in the neurons in the spinal cord is a new finding with significant implications in ALD and other disorders.
3. The neuronal uptake of dendrimers is significantly less in the spinal cord of wild type (WT) mice.

Studies revealed colocalization of dendrimer-Cy5 (D-Cy5) with Tuj 1 positive neurons in the spinal cord of *ABCD1* knockout ("KO") mice, while no clear D-Cy5 costaining was seen in healthy control mice. Pathological studies have shown axonal degeneration in *ACBD1* KO mice. The studies illustrate that dendrimer can be used as a vehicle for targeted delivery of therapeutic and/or diagnostic agent to the affected spinal neurons, with applications in the treatment and diagnosis of peroxisomal disorders and leukodystrophies, and molecular and clinical symptoms thereof.

### Example 2: The effects of size and surface properties on the in vivo pharmacokinetics of PAMAM dendrimers

Given the strong medical need for optimizing therapeutic delivery to overcome biological barriers, reduce off-site toxicity, and achieve efficacy, it is important to explore the *in vivo* mechanism of how these PAMAM dendrimers, with no targeting ligands, selectively localize in cells that mediate neuroinflammation. An *in vivo* rabbit model of CP, with features similar to CP in humans,(Saadani-Makki, et al., American Journal of Obstetrics and Gynecology 2008, 199(651), e651-657) was used to (1) characterize the impact of nanoparticle size on passage across an impaired BBB, (2) understand how dendrimer surface functionality dictates movement in the brain parenchyma and uptake by activated microglia, and (3) quantify dendrimer uptake and localization in the injured newborn brain as a function of disease severity.

### Materials and methods

### Preparation of dendrimer-Cy5 conjugates

Generation-4 PAMAM dendrimers, with hydroxyl (G4-OH), amine (G4-NH₂), and carboxylate (G3.5-COOH) end groups, were covalently conjugated with Cy5, a near-infrared (IR) imaging agent (details in supplemental material). Each dendrimer-Cy5 conjugate had 1-2 molecules of Cy5 on the surface of the dendrimer (5 wt%). The Cy5 conjugates were highly soluble in water, PBS buffer, and stable at physiological conditions G4-NH2 and G3.5-COOH are reference examples.

### Results

### Passage across an impaired BBB in CP kits is dependent on the physicochemical properties of dendrimers

The neuroinflammatory process results in injury to the surrounding oligodendrocytes and neurons, and disruption of the BBB at the site of injury (Li, et al., Proc. Natl. Acad. Sci. 2005, 102, 9936-9941; Stolp, et al., Cardiovascular Psychiatry and Neurology 2011, 2011, Article ID 469046), which can be chronic (de Vries, et al., Pharmacological Reviews 1997, 49, 143-155, Petty, et al., Progress in Neurobiology 2002, 68, 311-323). Following systemic administration, dendrimers will need to cross an impaired BBB to access the brain microenvironment. PAMAM dendrimers ranging from 3 nm to 14 nm were used to characterize the impaired BBB pore size in ischemic stroke, showing that a size of less than 11 nm is desirable to cross the impaired BBB in that model (Zheng, et al., Advanced Healthcare Materials 2014). Thus, experiments were carried out to determine how dendrimer size and molecular weight impact ability to cross the BBB in the CP model in regions of BBB breakdown.

The extent of extravasation, following systemic administration, into areas of injury in the brain of postnatal day 1 (PND1) rabbit kits with CP was evaluated for 70 kDa linear polymer dextran-FITC, and a hard spherical 20 nm polystyrene (PS) nanoparticle, and compared to that of G4-OH. The physicochemical properties of these compounds, including size and surface charge, are provided in Table 1.

**Table 1. Physicochemical properties of various platforms used to determine extravasation across the BBB and cellular uptake within the brain in CP kits.**

| **Platform** | **Physiological pH** | **MW ^{b} (kDa)** | **Size ± SEM ^{a} (nm)** | **Zeta potential ± SEM^{a} (mV)** |
|---|---|---|---|---|
| G4-OH | Neutral | 14.1 | 4.3 ± 0.2 | +4.5 ± 0.1 |
| G4-NH2 | Cationic | 14.1 | 3.9 ± 0.3 | +19.5 ±0.1 |
| G3.5-COOH | Anionic | 11.1 | 3.2 ± 0.4 | -12.2 ± 0.2 |
| 20 nm PS | Anionic | NA | 21 ± 1 | -23 ± 0.9 |
| Linear dextran | Neutral | 70.0 | 13.9 ± 1.3 | NA |
| G6-OH | Neutral | 58.0 | 6.7 ± 0.1 | 0.25 ± 0.4 |

| | | | | |
|---|---|---|---|---|
| ^{a} Hydrodynamic diameter (size) and surface charge (zetapotential) were measured using dynamic light scattering in PBS, pH 7.4 at room temperature. ^{b} Molecular weight was provided by the company, or determined using mass spectrometry for dendrimers. | | | | |

In regions of BBB impairment, dextran-FITC and 20 nm PS nanoparticles did not escape the blood vessel, or extravasate into the tissue 24h following systemic administration. On the other hand, G4-OH escaped the blood vessels and localized in cells in the periventricular region (PVR). In the brain of perfusion-fixed healthy animals, none of the materials showed measurable uptake or cellular localization up to 24h, since there was no BBB impairment.

### Dendrimer selectively localizes at sites of injury in the newborn brain

In the developing brain, new cell formation takes place, which is essential for normal development and maturation to occur. It is important to identify both the cells that do and do not take up dendrimers. There is BBB impairment and increased pro-inflammatory microglia expression in the PVR in CP kits.(Developmental Neuroscience 2011, 33, 231-240; Saadani-Makki, et al., J. Child Neurol. 2009, 24, 1179-1189).

At 4h after administration, G4-OH was present only in the *activated* glial ribbon of the PVR of animals with CP and in the choroid plexus, where there was significant blood vessel supply and cerebral spinal fluid (CSF)-blood exchange. In this model, it was shown that G4-OH only localized in this region of injury, and not in the subventricular zone (SVZ), where neuronal progenitor cells were present, or in the corpus callosum and cortex. This pattern of localization was observed even at later time points.

### Movement within the brain parenchyma is governed by nanoparticle size and surface functionality

After crossing an intact or impaired BBB, the brain extracellular space (ECS) is a conduit through which drug delivery platforms must diffuse. Activated microglia/astrocytes are often distributed diffusely throughout the brain in the ECS, and can be several microns from the nearest blood vessel (Bickel, et al., Advanced Drug Delivery Reviews 2001, 46, 247-279; Pawlik and Bing, Brain Res. 1981, 2008, 35-58; Schlageter, et al., Microvasc. Res. 1999, 58, 312-328). Even in regions of BBB impairment, both size and surface charge are critical to the ability of a drug delivery platform to cross the BBB,(Mayhan and Heistad, The American Journal of Physiology 1985, 248, H712-718; Pardridge, Journal of Cerebral Blood Flow and Metabolism: Official Journal of the International Society of Cerebral Blood Flow and Metabolism 2012, 32, 1959-1972) penetrate within the brain parenchyma,(Nance, et al., Science Translational Medicine 2012, 4, 149ra119) and reach diffuse cells often associated with CNS disorders to have maximum therapeutic effect.

It was found that, unlike G4-OH, 20 nm PS nanoparticles injected intraparenchymally in PND1 CP kits were not able to penetrate within the brain parenchyma away from the site of injection. This result was consistent to what has been previously demonstrated with unmodified (negatively charged) PS nanoparticles of sizes ranging from 40nm to 200nm (Nance, et al., Science Translational Medicine 2012, 4, 149ra119).

G4-OH and G4-NH2 were injected intraparenchymally in newborn kits with CP, and G4-OH was able to rapidly diffuse several millimeters away from the point of injection within 4h, and localize in cells only in regions of injury, whereas G4-NH2 remained trapped at the site of injection. Based on screening the brain using confocal imaging, PS nanoparticles and G4-NH2 were only able to follow routes of CSF flow, back along the injection track, into the subarachnoid space or into the choroid plexus, where they remained despite the presence of BBB impairment in the PVR.

### Dendrimer uptake and cellular localization in the injured newborn brain is a function of time and dendrimer surface functionality

It is important to understand the effect of dendrimer surface functionality on the dendrimer's ability to extravasate and localize in activated glial cells. The time dependence of G4-NH₂, G3.5-COOH, and G4-OH uptake in the brain was studied following systemic administration on PND1. These three dendrimers have approximately the same size and molecular weight, but different surface functionalities and zeta potentials at physiological pH (Table 1).

All animals were perfused with 1xPBS at time of sacrifice. G4-OH was able to extravasate and rapidly localize in activated microglia within 4h in regions of BBB impairment. At all the time points investigated in this study, G4-NH₂ remained trapped within blood vessels, likely due to charge interactions with negatively charged endothelial cell membranes (Jallouli, et al., International Journal of Pharmaceutics 2007, 344, 103-109). G3.5-COOH was not present in cells or blood vessels of the brain at 0.5h after injection, and was present in blood vessels at 4h and 24h, and in microglia cells at 24h. The delay in G3.5-COOH uptake in microglia cells compared to G4-OH uptake suggests that the neutral surface functionality on a dendrimer may be desirable for rapid escape from blood vessels.

In the confocal images, the varying pattern of intracellular distribution between G4-OH and G3.5-COOH was supported by previous intracellular trafficking studies, which showed G4-OH traffics to late lysosomes and G3.5-COOH sequesters in endosomes. G3.5-COOH could be useful for application in neuroinflammation since it also co-localizes in microglia, albeit in a delayed manner, and the different method of internalization compared to G4-OH could lead to targeting of specific intracellular pathways. G4-OH and G3.5-COOH localization at 24h after injection was also present in astrocytes in the PVR of CP kits.

In the brain of healthy PND1 kits, dendrimers did not cross the intact BBB, and remained localized within blood vessel structures, independent of dendrimer surface functionality. In CP kits, biodistribution in the heart, liver, and lungs, as well as clearance from the body via the kidneys, was similar for all G4 dendrimers studied. Based on previous biodistribution analysis of G4-OH, accumulation in the kidneys occurred up to 24h, as G4-OH was cleared from circulation (Lesniak, et al., Molecular Pharmaceutics 2013, 10, 4560-4571). There was no significant difference in biodistribution in the heart, liver, lungs and kidneys in control verse CP kits at this age.

The uptake and specific cellular localization of the dendrimer platforms can play a significant role in targeted delivery, especially if toxicity is of concern. Cationic PAMAM dendrimers have been shown to be taken up in the brain when administered intraparenchymally or intraventricularly (Albertazzi, et al., Molecular Pharmaceutics), but are also toxic at higher generations and higher concentrations through systemic and intranasal administration routes. This can lead to a negative effect on gene expression and the induction of autophagy due to increased intracellular reactive oxygen species generation (Win-Shwe, et al., Toxicol. Lett 2014, 228, 207-218; Wang, et al., Biomaterials 2014, 35, 7588-7597).

The inability of cationic dendrimers to diffuse within the brain parenchyma is also limiting, even if no toxicity for G4 or lower cationic dendrimers at low concentrations has been reported *in vivo* (Shcharbin, et al., Journal of Controlled Release 2014, 181, 40-42). It is important to emphasize that minimal or no G4-OH dendrimer uptake was seen in regions of healthy tissue, or in regions with new cell formation critical to normal brain development and function, which will reduce off-site toxicity and minimize long term negative impact. The ability of the neutral G4-OH to deliver drugs to activated glia, without associated toxicity, offers new avenues for targeted delivery.

### Semi-quantitative analysis of dendrimer uptake and cellular localization

The amount of dendrimer in the PVR of the brain, after perfusion, was quantified. The percent injected dose (%ID) of each dendrimer was calculated as the total amount of dendrimer in the brain (µg) over the total amount of brain tissue analyzed (g tissue). Peak uptake for all G4 dendrimers was observed at 4h after administration in PND1 CP kits, with a decrease in total amount in the brain by 24h (Figure 1A). G4-NH₂ was the most abundant in the brain at all time-points, yet was never present in cells within the parenchyma. G3.5-COOH and G4-OH had similar amounts in the brain at all time-points; however, the cellular localization of G3.5-COOH and G4-OH at each time point varied. The maximum %ID of G4-OH in the brain of kits with CP was 0.04%, compared to 0.003%ID of G4-OH in the brain of healthy control kits (> 10-fold overall uptake in the brain of CP kits). Importantly, the amount of G4-OH in the brain is 100-fold higher than that of a free drug (NAC), and the G4-OH is predominantly localized in target cells. The dose of dendrimer in this study is comparable to that of the dose of D-NAC that produced motor function improvement in CP showing that targeting the injured region of the brain, and specific cells, can lead to a profound effect (Kannan, et al., Science Translational Medicine 2012, 4(130), 130ra46; Mishra, et al., ACS Nano 2014, 8, 2134-2147).

Cellular localization of dendrimer was evaluated using semi-quantitative analysis of the confocal images. In recent years, a number of *in vitro* and *in vivo* studies have implicated microglial cells in the development of CP (Kannan, et al., Science Translational Medicine 2012, 4(130), 130ra46; Mallard, et al., Pediatric Research 2014, 75, 234-240). In the healthy brain, microglia are involved in surveillance functions, monitoring neuronal well-being (Billiards, et al., The Journal of Comparative Neurology 2006, 497, 199-208). Upon activation after an injury, microglia undergo a pronounced change in morphology from ramified to an amoeboid structure and proliferate, increasing in number (Perry, et al., Nature Reviews. Neurology 2010, 6, 193-201; Block, et al., Nature Reviews. Neuroscience 2007, 8, 57-69). The number of total microglia showed a 3.5-fold increase in the PVR of CP kits compared with healthy controls. However, the number of microglia in the cortex of CP kits remained comparable to that of healthy controls (Figure 1B). In the PVR of PND1 CP kits, the amoeboid population of microglia was 83% of the total microglia, compared to only 11% of total microglia in the PVR of healthy controls. In the rabbit model of CP, the number of microglia increases in the presence of inflammation, and there is an associated decrease in ramified "resting" microglia and an increase in amoeboid "activated" microglia. The microglia morphology in the cortex of both healthy and CP kits was predominantly ramified, with less than 4% of microglia classified as amoeboid.

Given the rapid uptake and previous use of G4-OH-drug conjugate in efficacy studies in CP (Kannan, et al., Science Translational Medicine 2012, 4(130), 130ra46), the cell specific change in localization of G4-OH over time was analyzed in the PVR and cortex of both healthy newborn kits and CP kits. The difference in co-localization of G4-OH over time corresponds to G4-OH movement from blood vessels at 0.5h to intracellular localization within microglia by 4h. Analysis of a representative region in the PVR showed co-localization of the G4-OH only with Iba-1 stained microglia, with no co-localization seen in the parenchyma. By analyzing a subset of 30µm thick sections within the PVR, the number of microglia that was positive for both G4-OH and Iba-1 at each time point was determined. The number of Iba-1+ microglia with G4-Cy5 increases in the PVR of kits with CP from 0.5h to 4h, and reaches a maximum of 90% of cells containing G4-OH. There was no uptake in microglia in the cortex of CP kits, or in the PVR or cortex of healthy control kits, due to the lack of BBB impairment. Based on previous cytokine data analysis in brains of kits with CP, it can be extrapolated that the dendrimer is localizing in "activated" microglia.

### Dendrimer is retained in the injured newborn brain

The uptake, long term retention, and release kinetics of dendrimer-drug conjugates will dictate both the timing of administration, as well as initial design of dendrimer-therapies. To determine if dendrimer is still present in microglia many days after administration, the retention of G4-OH in activated microglia in CP kits was measured. The longest average life expectancy of a CP kit without therapy is 9 days. At PND9 (8 days after systemic administration), G4-OH remained localized in microglia in the PVR. Unlike in PND1 kits, G4-OH was not present in blood vessels in PND9 CP kits, suggesting G4-OH that was not internalized by cells outside the brain tissue. The qualitative amount of G4-OH in the brain of PND9 kits was also reduced compared to 4h after systemic administration.

### Dendrimer uptake correlates to disease severity in newborn kits with CP

The toxicity of G4-OH, even at high doses, is minimal compared to cationic dendrimers, and the G4-OH dendrimer is cleared intact on the order of hours from blood circulation, and over 24-48h from the kidney (Lesniak, et al., Molecular Pharmaceutics 2013, 10, 4560-4571; Jones, et al., ACS Nano 2012, 6, 9900-9910; Jones, et al., Molecular Pharmaceutics 2012, 9, 1599-1611). G4-OH only accumulates in regions of injury where there is BBB impairment and cell activation, and not in normal healthy tissue or non-activated cells. Therefore, the extent of dendrimer uptake can be correlated to the extent of disease in the brain.

Animals were evaluated in a blinded manner for neurobehavioral measures, prior to dendrimer injection on PND1. A composite behavioral score was generated based on behavioral tests that were significantly different at PND1 between control kits and CP kits used in this study. Newborn kits with CP (n=18 total) were classified into the following categories: severe (n=6 kits, composite score 3-9), moderate (n=7 kits, composite score 10-14), and mild (n=5 kits, composite score 15-20). Normal healthy kits (n=8) had a composite behavioral score greater than 23. No kits with CP had a composite behavioral score greater than 20.

G4-OH was used to examine dendrimer uptake as a function of disease severity. In normal healthy control kits, minimal dendrimer accumulation (0.004%ID) was observed in the brain. In CP kits, up to 13-fold higher accumulation in kits with a severe phenotype, as assessed by composite behavioral score, was observed (Figure 2A). The amount of G4-OH uptake in the newborn CP brain was statistically greater in the severe group compared to normal (p<0.001) and mild kits (p<0.05). The G4-OH uptake in moderate and mild CP kits was significantly higher than healthy kits (p<0.005). However, there was no significant difference in the amount of G4-OH uptake in the severe kits compared to moderate kits, or in the moderate kits compared to mild kits.

Therefore it was determined if one could better delineate phenotype in the mild-moderate range based on dendrimer uptake in the CP brain. A Cy5-labeled, generation-6 dendrimer (G6-OH-Cy5) was used to evaluate uptake as a function of disease severity in CP kits (n=17 kits total) that fell into the mild (n=8) and moderate phenotype (n=9), with the same composite behavioral score ranges as described above. G6-OH has a longer circulation time compared to G4-OH (Kannan, et al., Journal of Internal Medicine 2014, 276(6), 579-617) and thus has greater uptake in the CP brain. However, G6-OH is still small enough in size and possesses neutral surface functionality (Table 1) to pass the impaired BBB and localize within microglial cells in the PVR of CP kits. A correlation (R2 = 0.51) between amount of G6-OH dendrimer in the brain (µg/g) and an increase in disease severity from mild to moderate was observed (Figure 2B). More importantly, the average amount of G6-OH uptake in moderate kits (1.33µg/g) was significantly greater (p<0.05) than the average amount of G6-OH uptake in mild kits (0.79µg/g). This trend was less when assessing individual behavioral scores (R2<0.50) in moderate CP kits that are statistically worse than mild CP kits. This shows that a comprehensive behavioral analysis, as performed clinically, is a more accurate assessment of disease severity than a single behavioral test.

### Example 3: Preparation and Characterization of Dendrimer-4-Phenyl Butyric Acid (D-PBA)

### Materials and Methods

### Materials and Reagents

Hydroxy functionalized enthylenediamine core generation 4.0 and 6.0 polyamidoamine (PAMAM) dendrimer (G4-OH; 64 hydroxyl end-groups and G6-OH; 256 hydroxyl end-groups) were purchased from Dendritech Inc. (Midland, MI, USA). N-acetylcystine (NAC), benzotriazol-1-yl-oxytripyrrolidinophosphonium hexafluorophosphate (PyBOP), 4-dimethyl aminopyridine (DMAP), N,N'-dicyclohexyl carbodiimide (DCC), 1-Ethyl-3-(3-dimethylaminopropyl)carbodiimide (EDCI), 3-mercaptopropanoic acid, tert-butyl 3-hydroxypropanoate and N,N'-dimethylformaimide (DMF) were purchased from Sigma-Aldrich (St Louis, MO, USA). 4-Phenyl butyrate was purchased from Cayman Chemicals (Michigan, MI, USA). Dialysis membranes (MWCO: 2kD) were purchased from Spectrum Laboratories Inc. (Ranco Dominguez, CA, USA).

### Results

### Preparation of Dendrimer-4-phenyl butyric acid (D-PBA)

4-phenyl butyric acid (PBA) was conjugated to hydroxyl-functionalized PAMAM dendrimer via a pH labile ester linkage. A propionyl linker was utilized as a spacer both to provide enough space for drug molecules on dendrimer surface and to facilitate their release. Since the attachment of linker is also based on an esterification reaction, a BOC group protection/deprotection strategy was followed to modify PBA molecules and then conjugation to dendrimer surface was performed for both 4th and 6th generation PAMAM dendrimers (Scheme 1).

Since PBA, in its neutralized form, is highly hydrophobic and water insoluble, feed ratio for drug conjugation reactions were kept low in order to obtain a conjugate which is both water soluble and has an enough multivalency with respect to multiple drug molecules attached to the same dendrimer molecule, with the aim of getting improved drug efficacy in both *in vitro* and *in vivo* studies.

### Neutralization of Sodium Phenyl Butyrate into 4-Phenyl Butyric Acid (PBA) (Compound 6)

Drug molecules were received in the form of sodium salt, where the carboxylic acid group in their structure is in anion form. In order to obtain the neutral form, these carboxylic acid groups were protonated via extraction by 1M HCl solution. Since the sodium salt of PBA is extremely water soluble, it (1 g, 5.34 mmol) was dissolved in a minimum amount of distilled water and then washed with 1M HCl (50 mL) and CH2Cl2 (50 mL) to collect the neutralized form of drug in organic phase. After removing excess water by NaSO4, organic phase was evaporated under vacuum and 4-phenyl butyric acid (PBA) (Compound **6**) was obtained as a white solid quantitatively (0.87 g).

### Synthesis of PBA-linker (Boc protected) (Compound 8)

Compound **6** (800.0 mg, 4.87 mmol) was dissolved in 10.0 mL anhydrous CH₂Cl₂, and then DMAP (238.0 mg, 1.95 mmol) and DCC (1.106 g, 5.36 mmol) were dissolved in 15.0 mL anhydrous CH₂Cl₂ and added in the round bottom flask. After the activation of carboxylic acid of Compound 6 by stirring the reaction mixture at 0°C for 30 minutes, tert-butyl 3-hydroxypropanoate (Compound 7) (1.08 mL, 7.31 mmol) diluted in 15.0 mL anhydrous CH₂Cl₂ was added and the reaction mixture was continued for 24 hours at room temperature (25 °C). Then all the volatiles were evaporated and the reaction crude mixture was purified by column chromatograph using silica gel as stationary phase and mixture of ethyl acetate/hexane (30:70) as eluent. The product was dried under vacuum and obtained as a white solid (Compound **8**) (1.075 g, 76% yield).

### Synthesis of PBA-linker (deprotected) (Compound 9)

Compound **8** (1.0 g, 3.42 mmol) was dissolved in 3.5 mL anhydrous CH₂Cl₂ and cooled down to 0°C. Then 10.0 mL TFA was added into the clear solution and the reaction mixture stirred at 0°C until the consumption of the starting material was observed on TLC. The crude mixture was purified by column chromatography using silica gel as stationary phase and mixture of ethyl acetate/hexane (40:60) as eluent. The product was dried under vacuum and obtained as a white solid (Compound 9) (0.7 g, 87% yield). High Resolution ESI-MS confirmed the molecular weight of the PBA-linker: Calculated: 236.264 (C₁₃H₁₆O₄); Found: 259.094 {M+Na+}.

### Synthesis of D-PBA

D-PBA conjugates were synthesized by the attachment of PBA-linker molecules to the surface of PAMAM dendrimers of both 4th and 6th generations (G4 and G6). The conjugation is based on the esterification reaction between carboxylic acid group of PBA-linker (deprotected) and hydroxyl groups of dendrimer. Table 3 summarizes the characteristic details of all conjugates synthesized as D-PBA.

### Representative procedure for large scale synthesis of D(G4)-PBA conjugate (Conjugate 2)

Compound **8** (330.8 mg, 1.40 mmol) was dissolved in 10.0 mL anydrous DMF and into this clear solution DMAP (85.5 mg, 0.70 mmol) and pyBOP (1.09 g, 2.10 mmol) dissolved in 15.0 mL anhydrous DMF were added. After stirring the reaction mixture at 0°C for 30 minutes, G4-PAMAM dendrimer (1 g, 0.07 mmol) dissolved in 5.0 mL anhydrous DMF was added and the reaction was left to continue for 2 days at room temperature (25 °C). Then the crude product was diluted with DMF and dialyzed against DMF to remove by-products and excess reactants, followed by H₂O to get rid of any organic solvent. Finally purified product was lyophilized and obtained as a white yellow solid (Conjugate 2) (1.24 g). The purity was subsequently verified on HPLC.

**Table 3. Synthesis and characterization details of prepared D-PBA conjugates**

| **No** | **D ^{a}** | **No. of PBA / D ^{a}** | **% of PBA (w/w)** | **MW ^{b} (g/mol)** | **Amount (mg)** |
|---|---|---|---|---|---|
| Conjugate 1 | G4 | 11.4 | 11.2 | 16703 | 237 |
| Conjugate 2 | G6 | 54 | 12.3 | 69835 | 190 |
| Conjugate 3 | G4 | 15 | 14.0 | 17489 | 1240 |
| Conjugate 4 | G6 | 51 | 12.1 | 69180 | 910 |

| | | | | | |
|---|---|---|---|---|---|
| ^{a} D: PAMAM Dendrimer ^{b} Molecular weight (MW) refers to the theoretical molecular weight of the conjugates. | | | | | |

### Characterization

The percentage loading of drug conjugated to dendrimer can be calculated from integration values of proton resonances belonging to amine protons of dendrimer emerging around 8.3-8.0 ppm, ester protons on both dendrimer and drug-linker molecule upon conjugation and on drug. Ester protons on PBA and propionyl linker appear at around 4.40 and 4.20 ppm as triplicates and the broad singlet at 4.02 ppm represents the ester protons formed upon reaction of hydroxyl groups on dendrimers with the drug-linker molecule. ¹H NMR spectra of D(G4)-OH, PBA-linker-deprotected, and D(G4)-PBA (500MHz) clearly showed extra peaks coming from the structure of drug-linker molecules compared to unreacted PAMAM dendrimer. Internal methylene bridge (CH₂) protons of PBA were seen to appear around 1.8 ppm as multiplicate, which can also be used to determine the number of drug molecules on dendrimer.

### In vitro Release Studies

Release profiles of PBA conjugated to PAMAM dendrimers of both 4th and 6th generations were investigated in three different environmental conditions. Since the linker is between an ester bond, the drug molecules on conjugates are expected to be released by hydrolysis in aqueous media less and comparably faster in acidic conditions. That is why conjugates were prepared as 2 mg/mL solutions in pH 7.4 PBS and pH 5.5 citrate buffer. Moreover, separate solutions for both conjugates were prepared in acidic media and porcine liver esterase was added as 1 unit per 1 µmol of ester in the conjugates. Catalytic activity of esterase was ensured by replenishing it at every other day during the release process. All the solutions were incubated at 37°C and samples were taken from those solutions at certain time points. Analysis of these samples using HPLC revealed the amount of drug released from the conjugate by calculating the amount of free drug quantitatively in the samples based on the AUC values of the peak of free drug.

According to obtained release profiles (Figures 3A-3B), it was clearly seen that there was an initial 30-40% drug release for both conjugates in the first few days, which then increased gradually over time. Up to 40 days, almost all PBA on G4 dendrimer was released, whereas for G6-PBA conjugate this value was about 65%.

### Example 4: Efficacy of dendrimer-4PBA in ALD/AMN patient-derived fibroblasts and macrophages

### Materials and Methods

### Cell Culture

Primary fibroblasts from male patients with either cerebral adrenoleukodystrophy (ALD) or adrenomyeloneuropathy (AMN) phenotype were thawed and plated in wells to grow for 4 days. On the 4th day cells were treated with various doses of Dendrimer-4phenylbutyrate (D4PBA) or free 4PBA and maintained in the culture, the treatment was refreshed on Day 7. Cells were harvested for analysis on Day 11. The C26:0, C22:0, and C20:0 very long chain fatty acid fraction of lysophosphatidyl choline (LysoPC) was measured in the harvested cells and the Lyso PC C26/C22 fraction was then calculated as a measure of impaired peroxisomal beta-oxidation.

### Results

As shown in FIG. 4, there was a dose dependent reduction of LysoPC C26/C22 ratio in the AMN cells, while a significant reduction was seen only at 300 micromolar D4PBA in the cerebral ALD cells. Free 4PBA had no effect on the Lyso PC C26/C22 ratio.

In a further experiment, peripheral blood mononucleocytes were derived from a cerebral ALD patient, two AMN and one control subject differentiated in culture using same protocol as above for D-NAC therapy. On day 3, cells were treated with various doses of D4-PBA, and then again on day 5, and day 7. On day 7, macrophages were again stimulated with very long chain fatty acids (VLCFA) as in the D-NAC macrophage study mentioned above. Cells were then harvested at 6h after stimulation.

As shown in FIGs. 5A-5C, all doses of D4PBA (30, 100, 300 micromolar) as well as free PBA at 300 micromolar reduced the VLCFA-induced TNF-alpha response both in controls and in the cerebral ALD and AMN patients. The results show that D-PBA improved peroxisomal beta oxidation and diminish the pro-inflammatory state of macrophages in ALD, and in AMN.

### Example 5: Preparation of hybrid dendrimer drug conjugates containing two drugs: NAC-Dendrimer-4PBA ((G4)-NAC&PBA)

### Results

Dendrimer conjugate that has two different drugs with two different linkers was successfully synthesized by attachment of PBA and NAC molecules to 4th generation PAMAM dendrimer sequentially. Scheme 7 represents all the reaction steps to obtain D-NAC&PBA conjugate.

Based on the nature of functional groups on both drug molecules and linkers, first pyridyl disulfide (PDS) containing propionyl linker was attached to dendrimer via an esterification reaction. Then as a second step, PBA-linker (deprotected) which was used for PBA conjugation, was reacted with hydroxyls on dendrimer with the same type of reaction via an ester bond, not to interfere with the carboxylic acid group on NAC molecules afterwards. Lastly, PDS units on the dendrimer were replaced with NAC molecules to form a disulfide bond via disulfide exchange reaction. All the intermediates were purified at each step of the whole synthesis pathway via both dialyses over DMF and precipitation in diethyl ether to give the final conjugate in its pure form.

Although this conjugate was synthesized by the attachment of PDS-linker to D-PDA conjugate which was prepared by the methodology mentioned above, the number of PDS-linkers conjugated to dendrimer was very few, which may be attributed to the hydrophobic nature of PBA molecules on dendrimer surface. However with this synthesis pathway, the dendrimer conjugate with two different drugs was successfully synthesized and obtained as a light yellow fluffy compound. Furthermore, these two different drugs can be released in different environmental conditions, and at different rates.

### Synthesis of D(G4)-PDS

2-pyridyldisulfide (PDP) group containing linker molecule (Compound **10**) was synthesized and then purified by column chromatography. Briefly, aldrithiol (2.07 g, 9.42 mmol) was dissolved in 20.0 mL MeOH, and then 3-mercapto propionic acid (410.5 µL, 4.71 mmol) was added in the round bottom flask. The reaction mixture was stirred for 24 hours at room temperature (25°C). Then all the volatiles were evaporated and the reaction crude was purified by column chromatography using silica gel as stationary phase and mixture of ethyl acetate/hexane (30:70) as eluent. The product was dried under vacuo and obtained as a yellow solid (Compound **10**) (868.0 mg, 80% yield).

Next, Compound **10** (290.4 mg, 1.27 mmol) was dissolved in 1.0 mL anhydrous DMF and into this clear solution DMAP (77.3 mg, 0.63 mmol) and pyBOP (988.2 mg, 1.90 mmol) dissolved in 3.0 mL anhydrous DMF were added. After stirring the reaction mixture at 0°C for 30 minutes, G4-PAMAM dendrimer (300.0 mg, 21.1 µmol) dissolved in 2.0 mL anhydrous DMF was added and the reaction was left to continue for 2 days at room temperature (25°C). Then the crude product was dialyzed against DMF to remove by-products and excess reactants, and then precipitated in diethyl ether to remove DMF. Finally purified product was re-dissolved in H2O, lyophilized and obtained as a yellow fluffy compound (355.0 mg). The theoretical MW of the product was 18440 gmol-1, and number of PDS/PAMAM was 20.

### Synthesis of D(G4)-PDS&PBA

Compound **9** (77.0 mg, 0.326 mmol) was dissolved in 2.0 mL anhydrous DMF and into this clear solution DMAP (19.9 mg, 0.163 mmol) and pyBOP (254.5 mg, 0.489 mmol) dissolved in 2.0 mL anhydrous DMF were added. After stirring the reaction mixture at 0oC for 30 minutes, D-PDS conjugate (300.0 mg, 16.3 µmol) dissolved in 1.0 mL anhydrous DMF was added and the reaction was left to continue for 2 days at room temperature (25°C). Then the crude product was dialyzed against DMF to remove by-products and excess reactants, and then precipitated in diethyl ether to get rid of DMF. Finally purified product was re-dissolved in H₂O, lyophilized and obtained as a light yellow fluffy compound (312.0 mg). The theoretical MW of the product was 21060 g/mol, and number of PBA/PAMAM was 12.

### Synthesis of D(G4)-NAC&PBA

D-PDS and PBA conjugate (300.0 mg, 14.2 µmol) was dissolved in 3.0 mL anhydrous DMF, and then NAC (58.1 mg, 0.356 mmol) dissolved in 2.0 mL anhydrous DMF was added in the round bottom flask. The reaction mixture was stirred for 24 hours at room temperature (25°C). Then all the volatiles were evaporated and the reaction crude was purified by dialysis against DMF to remove by-products and excess reactants, and then followed by water to get rid of all organic solvents. Lastly it was lyophilized and obtained as a light yellow fluffy compound (285.0 mg). The theoretical MW of the product was 22100 g/mol, % of PBA by weight: 8.9, # of NAC/PAMAM: 20, % of NAC by weight: 14.8.

### Characterization

1H NMR spectra of PAMAM dendrimer without any conjugation, the intermediates during the synthesis pathway, and the final conjugate as D-NAC&PBA were analyzed. Upon attachment of PDS-propionyl linker, the aromatic protons of PDS ring show up around 7-8 ppm, some of which were overlapped with the internal amine protons of PAMAM dendrimer at around 8 ppm. Ester protons formed on dendrimer via linker attachment can be detected clearly at 4.02 ppm, whose integration values were utilized for the calculation of number of PDS groups conjugated to dendrimer surface.

Next, PBA drug was inserted to conjugate structure via the esterification reaction through a propionyl linker it was already attached. After several purification steps, increase in proton signals at aromatic region at around 7.0-7.5 ppm and additional ester protons appearing as triplates in the upper region of ester protons of dendrimer clearly proves the conjugation of PBA-linker molecules. Apart from the integration values of these signals, integration value of internal CH₂'s of PBA at around 1.8 ppm can also be used for calculating PBA payload per dendrimer.

Lastly, NAC molecules were conjugated to dendrimer by replacing PDS ring on the D-PDS&PBA conjugate. Upon disulfide exchange reaction, a remarkable decrease in the integration values around aromatic region indicates that this replacement reaction took place. Moreover, appearance of the broad singlet at 1.86 ppm refers to methyl protons of NAC, whose integration is consistent with the number of PDS groups on conjugate before.

The appearance of new peaks and shifts of protons at the reaction region in the structure clearly proves the successful synthesis of D-NAC&PBA conjugate with two different linkers, together with the integration values of characteristic peaks belonging to both dendrimer and individual drug molecules.

### Example 6: Effect of Dendrimer-NAC conjugates on ALD patient derived macrophages

### Materials and Methods

### Cell culture

Peripheral blood monocytes were derived from patient and control blood immediately following venous blood draw, using double gradient centrifugation. M1-like adherent macrophages were differentiated in DMEM (ThermoFisher, Waltham, MA), 10% FBS (Thermo Fisher, Waltham, MA), 10.000U/mL PenStrep (Corning, Pittsburgh, PA), 1% Glutamine (Thermo Fisher, PA), 1% NEAA (Thermofischer, Waltham, MA), GM-CSF (Thermofischer, Waltham, MA) and IL-4 (Thermo Fisher, Waltham, MA) for 7 days with media replaced on days 3, 5 and 7.

Macrophages were stimulated with 30 µM very long chain fatty acids (VLCFA) (C24:0 and C26:0 suspended in 10% heat inactivated FBS (Thermo Fisher, Waltham, MA)) and concomitantly treated with various doses of Dendrimer-NAC. Cell and supernatant were harvested 6h after stimulation and treatment.

### Assays

Commercially available assays were performed to determine levels of TNFα (Cayman, Ann Arbor, MA), Glutamate (Cayman, Ann Arbor, MA) and Glutathione (Abeam, Cambridge, MA). Spectrophotometer measurement was performed using a Spectramax^{®} M5 from Molecular Devices (Sunnyvale, CA).

### Results

Dendrimer-NAC conjugates show dose-dependent efficacy in attenuating TNFα expression (inflammation) and glutamate secretion (excitotoxicity) in cALD patient-derived macrophages, without affecting the cells from healthy or AMN patients. As shown below in FIGs.6A-6D and FIGs.7A-7D, VLCFA stimulation resulted in a significant increase in TNF-alpha and glutamate levels in macrophages of AMN and cerebral ALD patients but not in controls or ALD heterozygotes. Concomitant Dendrimer-NAC (D-NAC) therapy reduced the TNF-alpha response at 30 and 100 micromolar but not at 300 micromolar concentration in AMN patient cells, while there was a clear dose response in cerebral ALD (cALD) macrophages. Glutamate release was reduced in a dose dependent manner in both AMN and cerebral ALD macrophages. Cerebral ALD macrophages showed a dramatically reduced total glutathione level after VLCFA stimulation which was increased in a dose dependent manner with D-NAC (FIGs.8A-8D).

### Example 7: Synthesis of Dendrimer-Bezafibrate (D-BEZA)

### Materials and Methods

Bezafibrate (BEZA) was conjugated to hydroxyl functionalized PAMAM dendrimer via a pH labile ester linkage. Same strategy was applied for the synthesis of bezafibrate-PAMAM conjugates as in the sythesis of D-PBA conjugates mentioned above. This conjugation depends on the same BOC group protection/deprotection strategy for the sequential esterification reactions first to attach the linker to bezafibrate, and then conjugate the drug-linker compound to dendrimer surface. Same propionyl linker was utilized as a spacer here as well both to provide enough space for drug molecules on dendrimer surface and to facilitate their release. Synthesis of conjugates with bezafibrate was performed for both 4th and 6th generation PAMAM dendrimers (Scheme 8).

Since bezafibrate is very hydrophobic and water insoluble like PBA drug, feed ratio for bezafibrate conjugation reactions were kept low as well in order to obtain a conjugate which is both water soluble and has an enough multivalency degree referring to drug payload with the aim of getting a better drug efficacy for both in vitro and in vivo studies.

### Synthesis of BEZA-linker (Boc protected) (Compound 11)

Bezafibrate (800.0 mg, 2.21 mmol) was dissolved in 10.0 mL anhydrous CH2Cl2, and then DMAP (108.0 mg, 0.88 mmol) and DCC (501.8 mg, 2.43 mmol) were dissolved in 15.0 mL anhydrous CH2Cl2 and added in the round bottom flask. After the activation of carboxylic acid of drug by stirring the reaction mixture at 0°C for 30 minutes, tert-butyl 3-hydroxypropanoate (2) (0.49 mL, 3.32 mmol) diluted in 15.0 mL anhydrous CH2Cl2 was added and the reaction mixture was continued for 24 hours at room temperature (25 °C). Then all the volatiles were evaporated and the reaction crude was purified by column chromatograph using silica gel as stationary phase and mixture of ethyl acetate/hexane (30:70) as eluent. The product was dried under vacuo and obtained as a white solid (Compound **11**) (1.04 g, 96% yield).

### Synthesis of BEZA-linker (deprotected) (Compound 12)

Compound **11** (1.0 g, 2.04 mmol) was dissolved in 3.5 mL anhydrous CH2Cl2 and cooled down to 0oC. Then 6.10 mL TFA was added into the clear solution and the reaction mixture let to stir at 0oC until the consumption of the starting material was observed on TLC. The crude was purified by column chromatograph using silica gel as stationary phase and mixture of ethyl acetate/hexane (40:60) as eluent. The product was dried under vacuo and obtained as a white solid (Compound **12**) (0.88 g, 88% yield). High Resolution ESLMS confirmed the molecular weight of the BEZA-linker: Calculated: 434.137 (C22H25ClNO6); Found: 434.138 M+ 11, 456.121 {M+Na+1}

### Synthesis of D-BEZA

D-BEZA conjugates were synthesized by the attachment of BEZA-linker molecules to the surface of PAMAM dendrimers of both 4^{th} and 6^{th} generations. The conjugation is based on the esterification reaction between carboxylic acid group of BEZA -linker (deprotected) and hydroxyl groups of dendrimer. Table 4 summarizes the characteristic details of all conjugates synthesized as D-BEZA.

**Table 4. Synthesis and characterization details of prepared D-BEZA conjugates**

| **No** | **D ^{a}** | **No. of BEZA / D ^{a}** | **% of BEZA (w/w)** | **MW ^{b} (g/mol)** | **Amount (mg)** |
|---|---|---|---|---|---|
| Conjugate 6 | G4 | 10 | 19.7 | 18374 | 120 |
| Conjugate 7 | G6 | 42 | 20.1 | 75515 | 215 |
| Conjugate 8 | G4 | 8 | 16.5 | 17542 | 950 |
| Conjugate 9 | G6 | 28 | 14.5 | 69693 | 1090 |

| | | | | | |
|---|---|---|---|---|---|
| ^{a} D: PAMAM Dendrimer ^{b} Molecular weight (MW) refers to the theoretical molecular weight of the conjugates. | | | | | |

### Representative procedure for large scale synthesis of D(G4)- BEZA conjugate (Conjugate 8)

Compound **12** (610.0 mg, 1.40 mmol) was dissolved in 10.0 mL anhydrous DMF and into this clear solution DMAP (85.5 mg, 0.70 mmol) and pyBOP (1.09 g, 2.10 mmol) dissolved in 15.0 mL anhydrous DMF were added. After stirring the reaction mixture at 0 °C for 30 minutes, G4-PAMAM dendrimer (1 g, 0.07 mmol) dissolved in 5.0 mL anhydrous DMF was added and the reaction was left to continue for 2 days at room temperature (25°C). Then the crude product was diluted with DMF and dialyzed against DMF to remove by-products and excess reactants, followed by H2O to get rid of any organic solvent. Finally purified product was lyophilized and obtained as a white yellow solid (Conjugate **8**) (0.95 g). The theoretical MW of product was 17542 gmol-1, and No. of BEZA /PAMAM was 8% of BEZA by weight: 16.5.

### Characterization

The percentage loading of drug conjugated to dendrimer can be calculated from integration values of proton resonances belonging to amide protons of dendrimer emerging around 8.3-8.0 ppm, ester protons on both dendrimer and drug-linker molecule upon conjugation and on drug. Ester protons of propionyl linker appeared at around 4.40 ppm as multiplicate , and another multiplicate at around 4.00 ppm represented the ester protons formed upon reaction of hydroxyl groups on dendrimers with the drug-linker molecule. ¹H NMR spectra of Bezafibrate, BEZA-linker-Boc protected, and BEZA-linker-deprotected (CDCl3, 500MHz) showed extra peaks coming from the structure of drug-linker molecules compared to unreacted PAMAM dendrimer. Methyl (CH₃) protons of BEZA were seen to appear around 1.4 ppm as broad singlet, which can also be used to determine the number of drug molecules on dendrimer.

## Claims

1. A dendrimer complex, for use in a method of treatment of a peroxisomal disorder in a subject, wherein the complex comprises generation 4-10 poly(amidoamine) (PAMAM) hydroxyl-terminated dendrimers complexed, conjugated or intra-molecularly dispersed or encapsulated with at least one therapeutic or prophylactic agent, wherein the agent is for the treatment of a peroxisomal disorder, and wherein the complex targets microglia.

2. A dendrimer complex for use in a method according to claim 1, wherein the peroxisomal disorder:
is a peroxisome biogenesis disorder;
comprises effects on the growth or maintenance of the myelin sheath that insulates nerve cells; or
is a leukodystrophy.

3. A dendrimer complex for use in a method according to claim 2, wherein the leukodystrophy is selected from the group consisting of 18q Syndrome with deficiency of myelin basic protein, Acute Disseminated Encephalomyelitis (ADEM), Acute Disseminated Leukoencephalitis, Acute Hemorrhagic Leukoencephalopathy, X-Linked Adrenoleukodystrophy (ALD), Adrenomyeloneuropathy (AMN), Aicardi-Goutieres Syndrome, Alexander Disease, Adult-onset Autosomal Dominant Leukodystrophy (ADLD), Autosomal Dominant Diffuse Leukoencephalopathy with neuroaxonal spheroids (HDLS), Autosomal Dominant Late-Onset Leukoencephalopathy, Childhood Ataxia with diffuse CNS Hypomyelination (CACH or Vanishing White Matter Disease), Canavan Disease, Cerebral Autosomal Dominant Arteropathy with Subcortical Infarcts and Leukoencephalopathy (CADASIL), Cerebrotendinous Xanthomatosis (CTX), Craniometaphysical Dysplasia with Leukoencephalopathy, Cystic Leukoencephalopathy with RNASET2, Extensive Cerebral White Matter abnormality without clinical symptoms, Familial Adult-Onset Leukodystrophy manifesting as cerebellar ataxia and dementia, Familial Leukodystrophy with adult onset dementia and abnormal glycolipid storage, Globoid Cell Leukodystrophy (Krabbe Disease), Hereditary Adult Onset Leukodystrophy simulating chronic progressive multiple sclerosis, Hypomyelination with Atrophy of the Basal Ganglia and Cerebellum (HABC), Hypomyelination, Hypogonadotropic, Hypogonadism and Hypodontia (4H Syndrome), Lipomembranous Osteodysplasia with Leukodystrophy (Nasu Disease), Metachromatic Leukodystrophy (MLD), Megalencephalic Leukodystrophy with subcortical Cysts (MLC), Neuroaxonal Leukoencephalopathy with axonal spheroids (Hereditary diffuse leukoencephalopathy with spheroids - HDLS), Neonatal Adrenoleukodystrophy (NALD), Oculodetatoldigital Dysplasia with cerebral white matter abnormalities, Orthochromatic Leukodystrophy with pigmented glia, Ovarioleukodystrophy Syndrome, Pelizaeus Merzbacher Disease (X-linked spastic paraplegia), Refsum Disease, Sjogren-Larssen Syndrome, Sudanophilic Leukodystrophy, Van der Knaap Syndrome (Vacuolating Leukodystrophy with Subcortical Cysts or MLC), Vanishing White Matter Disease (VWM) or Childhood ataxia with diffuse central nervous system hypomyelination, (CACH), Infantile Refsum Disease, and Leukoencephalopathy with brainstem and spinal cord involvement and lactate elevation (LBSL)/DARS2 Leukoencephalopathy.

4. A dendrimer complex for use in a method according to any one of claims 1-3, wherein the subject is a child between birth and 18 years of age.

5. A dendrimer complex for use in a method according to any one of claims 1-4, wherein the PAMAM dendrimers are generation 6 PAMAM dendrimers.

6. A dendrimer complex for use in a method according to any one of claims 1-5, wherein the dendrimers are conjugated to the therapeutic agent in an amount effective to reduce or inhibit peroxisomal β-oxidation, glutamate secretion, one or more pro-inflammatory cytokines, or any combination thereof in the activated microglia.

7. A dendrimer complex for use in a method according to any one of claims 1-6, wherein the dendrimers are conjugated to one or more therapeutic agents in an amount effective to reduce or otherwise alleviate oxidative stress, neuroinflammation, long chain fatty acid production, loss of motor function, or a combination thereof; to promote, increase, or improve peroxisome proliferation, very long chain fatty acid removal, motor function, ABCD2 expression, enzymes mutated or deficient in peroxisomal disorders, or a combination thereof.

8. A dendrimer complex for use in a method according to any one of claims 1-7, wherein the therapeutic agent is:
an anti-inflammatory or antioxidant; optionally
wherein the anti-inflammatory is selected from the group consisting of steroidal anti-inflammatory agents, non-steroidal anti-inflammatory agents, and gold compound anti-inflammatory agents; further optionally
wherein the anti-inflammatory is VBP15;
a wildtype copy of an enzyme that is otherwise mutated or deficient in a peroxisomal disorder or leukodystrophy or a nucleic acid encoding the enzyme; optionally
wherein the enzyme is galactosylceramidase (GALC), Aspartoacylase (ASPA) or Arylsulfatase A (ARSA);
a thyroid hormone or a thyromimetic;
optionally
wherein the thyroid hormone is a natural or synthetic triiodothyronine (T3), its prohormone thyroxine (T4), or a mixture thereof; or
wherein the thyromimetic is sobetirome;
4-phenyl butyrate, benzafibrate, N-acetylcysteine, pioglitazone, or Vitamin E;
an agent that improves redox homeostasis and/or mitochondrial respiration;
an agent that reduces or reverses bioenergetic failure, axonal degeneration, and/or
associated locomotor disabilities, or a combination thereof; optionally
wherein the agent is Resveratrol.

9. A dendrimer complex for use in a method according to any one of claims 1-8 wherein the dendrimer is conjugated to two different therapeutic agents; optionally
wherein the first therapeutic agent is an anti-inflammatory or antioxidant; and
wherein the second therapeutic agent is selected from the group consisting of N-acetylcysteine, 4-phenylbutyrate, bezafibrate, thyroid hormone (T3), sobetirome, pioglitazone, resveratrol, VBP15, Vitamin E, erucic acid, biotin, Coenzyme Q10, clemastine, galactosylceramidase (GALC), and Arylsulfatase A (ARSA).

10. A dendrimer complex for use in a method according to any one of claims 1-9, wherein the therapeutically active agent is for localizing and targeting Neuron-specific class III beta-tubulin (TUJ-1) positive spinal neurons.

11. The dendrimer complex for use in a method of any one of claims 1-10, wherein the dendrimer complex is formulated in a suspension, emulsion, or solution; and/or
is administered to the subject in a time period selected from the group consisting of: every other day, every three days, every 4 days, weekly, biweekly, monthly, and bimonthly.

12. A dendrimer composition comprising dendrimers complexed, covalently attached or intra-molecularly dispersed or encapsulated with a therapeutic agent, wherein the dendrimers are generation 4-6 poly(amidoamine) (PAMAM) hydroxyl-terminated dendrimers and the therapeutic agent is Coenzyme Q10, clemastine, VBP15, wildtype copies of galactosylceramidase (GALC) or Arylsulfatase A (ARSA) or a nucleic acid encoding GALC or ARSA, a thyroid hormone, a thyromimetic, 4-phenyl butyrate, erucic acid, pioglitazone, benzafibrate, or any combination thereof.

13. The dendrimer composition of claim 12 wherein the thyroid hormone is natural or synthetic triiodothyronine (T3), its prohormone thyroxine (T4), or a mixture thereof.

14. The dendrimer composition of claim 12 wherein the thyromimetic is sobetirome.

## Patentansprüche

1. Dendrimerkomplex zur Verwendung in einem Verfahren zur Behandlung einer peroxisomalen Störung bei einem Subjekt, wobei der Komplex Poly (amidoamin) (PAMAM) - Dendrimere der Generation 4-10 mit endständigen Hydroxylgruppen umfasst, die mit mindestens einem therapeutischen oder prophylaktischen Mittel komplexiert, konjugiert oder intramolekular dispergiert oder eingekapselt sind, wobei das Mittel der Behandlung einer peroxisomalen Störung dient, und wobei der Komplex auf Mikroglia abzielt.

2. Dendrimerkomplex zur Verwendung in einem Verfahren nach Anspruch 1, wobei die peroxisomale Störung:
eine Peroxisom-Biogenese-Störung ist;
Auswirkungen auf das Wachstum oder den Erhalt der Myelinscheide umfasst, die Nervenzellen isoliert; oder
eine Leukodystrophie ist.

3. Dendrimerkomplex zur Verwendung in einem Verfahren nach Anspruch 2, wobei die Leukodystrophie ausgewählt ist aus der Gruppe, bestehend aus 18q-Syndrom mit Mangel an basischem Myelinprotein, akuter disseminierter Enzephalomyelitis (ADEM), akuter disseminierter Leukenzephalitis, akuter hämorrhagischer Leukenzephalopathie, X-chromosomaler Adrenoleukodystrophie (ALD), Adrenomyeloneuropathie (AMN), Aicardi-Goutières-Syndrom, Alexander-Krankheit, autosomal-dominanter Leukodystrophie (ADLD) mit Beginn im Erwachsenenalter, autosomal-dominanter diffuser Leukenzephalopathie mit neuroaxonalen Sphäroiden (HDLS), spät einsetzender autosomal-dominanter Leukenzephalopathie, Ataxie im Kindesalter mit diffuser Hypomyelinisierung des ZNS (CACH oder Vanishing-White-Matter-Erkrankung), Canavan-Krankheit, zerebraler autosomal-dominanter Arteropathie mit subkortikalen Infarkten und Leukenzephalopathie (CADASIL), zerebrotendinöse Xanthomatose (CTX), kraniometaphysischer Dysplasie mit Leukenzephalopathie, zystische Leukenzephalopathie mit RNASET2, weitreichender Anomalie der zerebralen weißen Substanz ohne klinische Symptome, familiärer Leukodystrophie mit Beginn im Erwachsenenalter, die sich als zerebelläre Ataxie und Demenz manifestiert, familiärer Leukodystrophie mit beginnender Demenz im Erwachsenenalter und abnormaler Glykolipidspeicherung, Globoidzell-Leukodystrophie (Krabbe-Krankheit), hereditärer Leukodystrophie mit Beginn im Erwachsenenalter, die eine chronische progressive Multiple Sklerose simuliert, Hypomyelinisierung mit Atrophie der Basalganglien und des Kleinhirns (HABC), Hypomyelinisierung, Hypogonadotropie, Hypogonadismus und Hypodontie (4H-Syndrom), lipomembranöser Osteodysplasie mit Leukodystrophie (Nasu-Krankheit), metachromatischer Leukodystrophie (MLD), megalenzephaler Leukodystrophie mit subkortikalen Zysten (MLC), Neuroaxonale Leukenzephalopathie mit axonalen Sphäroiden (hereditäre diffuse Leukenzephalopathie mit Sphäroiden - HDLS), neonataler Adrenoleukodystrophie (NALD), okulodetatoldigitaler Dysplasie mit Anomalien der zerebralen weißen Substanz, orthochromatischer Leukodystrophie mit pigmentierter Glia, Ovarioleukodystrophie-Syndrom, Pelizaeus-Merzbacher-Krankheit (X-chromosomale spastische Paraplegie), Refsum-Krankheit, Sjögren-Larsson-Syndrom, sudanophiler Leukodystrophie, Van-der-Knaap-Syndrom (vakuolisierende Leukodystrophie mit subkortikalen Zysten oder MLC), Vanishing-White-Matter-(VWM)-Erkrankung oder Ataxie im Kindesalter mit diffuser Hypomyelinisierung des Zentralnervensystems (CACH), infantiler Refsum-Krankheit und Leukenzephalopathie mit Hirnstamm- und Rückenmarksbeteiligung und Laktaterhöhung (LBSL)/DARS2 Leukenzephalopathie.

4. Dendrimerkomplex zur Verwendung in einem Verfahren nach einem der Ansprüche 1-3, wobei das Subjekt ein Kind zwischen Geburt und 18 Jahren ist.

5. Dendrimerkomplex zur Verwendung in einem Verfahren nach einem der Ansprüche 1-4, wobei die PAMAM-Dendrimere PAMAM-Dendrimere der Generation 6 sind.

6. Dendrimerkomplex zur Verwendung in einem Verfahren nach einem der Ansprüche 1-5, wobei die Dendrimere an das therapeutische Mittel in einer Menge konjugiert sind, die wirksam ist, um eine peroxisomale β-Oxidation, eine Glutamatsekretion, ein oder mehrere proinflammatorische Zytokine oder eine beliebige Kombination davon in der aktivierten Mikroglia zu reduzieren.

7. Dendrimerkomplex zur Verwendung in einem Verfahren nach einem der Ansprüche 1-6, wobei die Dendrimere mit einem oder mehreren therapeutischen Mitteln in einer Menge konjugiert sind, die wirksam ist, um oxidativen Stress, eine Neuroinflammation, eine Produktion langkettiger Fettsäuren, einen Verlust von motorischer Funktion oder eine Kombination davon zu reduzieren oder anderweitig zu lindern; um eine Peroxisom-Proliferation, eine Entfernung sehr langkettiger Fettsäuren, motorische Funktion, eine ABCD2-Expression, Enzyme, die bei peroxisomalen Störungen mutiert oder defizient sind, oder einer Kombination davon zu fördern, zu erhöhen oder zu verbessern.

8. Dendrimerkomplex zur Verwendung in einem Verfahren nach einem der Ansprüche 1-7, wobei das therapeutische Mittel Folgendes ist:
ein Entzündungshemmer oder ein Antioxidans;
wobei optional der Entzündungshemmer ausgewählt ist aus der Gruppe, bestehend aus steroidalen Entzündungshemmern, nicht-steroidalen Entzündungshemmern und Entzündungshemmern mit Goldverbindung;
wobei optional weiter der Entzündungshemmer VBP15 ist;
eine Wildtyp-Kopie eines Enzyms, das bei einer peroxisomalen Störung oder Leukodystrophie anderweitig mutiert oder defizient ist, oder eine Nukleinsäure, die das Enzym kodiert;
wobei optional das Enzym Galactosylceramidase (GALC), Aspartoacylase (ASPA) oder Arylsulfatase A (ARSA) ist;
ein Schilddrüsenhormon oder ein Thyromimetikum;
wobei optional das Schilddrüsenhormon ein natürliches oder synthetisches Triiodthyronin (T3), sein Prohormon Thyroxin (T4) oder eine Mischung davon ist; oder
wobei das Thyromimetikum Sobetirom ist;
4-Phenylbutyrat, Benzafibrat, N-Acetylcystein, Pioglitazon oder Vitamin E;
ein Mittel, das Redox-Homöostase und/oder mitochondriale Atmung verbessert;
ein Mittel, das bioenergetisches Versagen, axonale Degeneration und/oder damit verbundene lokomotorische Behinderungen oder eine Kombination davon reduziert oder umkehrt;
wobei optional das Mittel Resveratrol ist.

9. Dendrimerkomplex zur Verwendung in einem Verfahren nach einem der Ansprüche 1-8, wobei das Dendrimer mit zwei verschiedenen therapeutischen Mitteln konjugiert ist;
wobei optional das erste therapeutische Mittel ein Entzündungshemmer oder ein Antioxidans ist; und
wobei das zweite therapeutische Mittel ausgewählt ist aus der Gruppe, bestehend aus N-Acetylcystein, 4-Phenylbutyrat, Bezafibrat, Schilddrüsenhormon (T3), Sobetirom, Pioglitazon, Resveratrol, VBP15, Vitamin E, Erucasäure, Biotin, Coenzym Q10, Clemastin, Galactosylceramidase (GALC) und Arylsulfatase A (ARSA).

10. Dendrimerkomplex zur Verwendung in einem Verfahren nach einem der Ansprüche 1-9, wobei das therapeutisch aktive Mittel zum Lokalisieren und Targeting von Neuronen-spezifischen Beta-Tubulin (TUJ-1)-positiven spinalen Neuronen der Klasse III dient.

11. Dendrimerkomplex zur Verwendung in einem Verfahren nach einem der Ansprüche 1-10, wobei der Dendrimerkomplex in einer Suspension, Emulsion oder Lösung formuliert ist; und/oder
dem Subjekt in einem Zeitraum verabreicht wird, der ausgewählt ist aus der Gruppe, bestehend aus: jeden zweiten Tag, alle drei Tage, alle 4 Tage, wöchentlich, zweiwöchentlich, monatlich und zweimonatlich.

12. Eine Dendrimer-Zusammensetzung, umfassend Dendrimere, die mit einem therapeutischen Mittel komplexiert, kovalent gebunden oder intramolekular dispergiert oder eingekapselt sind, wobei die Dendrimere Poly(amidoamin) (PAMAM)-Dendrimere der Generation 4-6 mit endständigen Hydroxylgruppen sind und das therapeutische Mittel Coenzym Q10, Clemastin, VBP15, Wildtyp-Kopien von Galactosylceramidase (GALC) oder Arylsulfatase A (ARSA) oder eine Nukleinsäure, die GALC oder ARSA kodiert, ein Schilddrüsenhormon, ein Thyromimetikum, 4-Phenylbutyrat, Erucasäure, Pioglitazon, Benzafibrat oder eine beliebige Kombination davon ist.

13. Dendrimer-Zusammensetzung nach Anspruch 12, wobei das Schilddrüsenhormon natürliches oder synthetisches Triiodthyronin (T3), sein Prohormon Thyroxin (T4) oder eine Mischung davon ist.

14. Dendrimer-Zusammensetzung nach Anspruch 12, wobei das Thyromimimetikum Sobetirom ist.

## Revendications

1. Complexe de dendrimère, destiné à être utilisé dans un procédé de traitement d'un trouble peroxysomal chez un sujet, dans lequel le complexe comprend des dendrimères de poly(amidoamine) (PAMAM) à terminaison hydroxyle de génération 4 à 10 complexés, conjugués ou dispersés de manière intramoléculaire ou encapsulés avec au moins un agent thérapeutique ou prophylactique, dans lequel l'agent est destiné au traitement d'un trouble peroxysomal, et dans lequel le complexe cible la microglie.

2. Complexe de dendrimère destiné à être utilisé dans un procédé selon la revendication 1, dans lequel le trouble peroxysomal :
est un trouble de la biogenèse des peroxysomes ;
comprend des effets sur la croissance ou le maintien de la gaine de myéline qui isole les cellules nerveuses ; ou
est une leucodystrophie.

3. Complexe de dendrimère destiné à être utilisé dans un procédé selon la revendication 2, dans lequel la leucodystrophie est choisie dans le groupe constitué du syndrome 18q avec déficit en protéine basique de la myéline, de l'encéphalomyélite aiguë disséminée (ADEM), de la leucoencéphalite aiguë disséminée, de la leucoencéphalopathie hémorragique aiguë, de l'adrénoleucodystrophie liée au sexe (ALD), de l'adrénomyéloneuropathie (AMN), du Syndrome d'Aicardi-Goutières, de la maladie d'Alexander, de la leucodystrophie autosomique dominante (ADLD) de l'adulte, de la leucoencéphalopathie diffuse autosomique dominante avec sphéroïdes neuroaxonaux (HDLS), de la leucoencéphalopathie autosomique dominante tardive, de l'ataxie infantile avec hypomyélinisation diffuse du SNC (CACH ou Vanishing White Matter Disease), de la maladie de Canavan, de l'artéropathie cérébrale autosomique dominante avec infarctus sous-corticaux et leucoencéphalopathie (CADASIL), de la xanthomatose cérébrotendineuse (CTX), de la dysplasie craniométaphysique avec leucoencéphalopathie, de la leucoencéphalopathie kystique avec RNASET2, de l'anomalie cérébrale étendue de la substance blanche sans symptômes cliniques, de la leucodystrophie familiale de l'adulte se manifestant par une ataxie cérébelleuse et une démence, de la leucodystrophie familiale avec démence de l'adulte et stockage anormal des glycolipides, de la leucodystrophie des cellules globoïdes (maladie de Krabbe), de la leucodystrophie héréditaire de l'adulte simulant une sclérose en plaques progressive chronique, de l'hypomyélinisation avec atrophie des ganglions de la base et cervelet (HABC), de l'hypomyélinisation, de l'hypogonadotrope, de l'hypogonadisme et de l'hypodontie (syndrome 4H), de l'ostéodysplasie lipomembraneuse avec leucodystrophie (maladie de Nasu), de la leucodystrophie métachromatique (MLD), de la leucodystrophie mégalencéphalique avec kystes sous-corticaux (MLC), de la leucoencéphalopathie neuroaxonale avec sphéroïdes axonaux (Leucoencéphalopathie diffuse héréditaire avec sphéroïdes - HDLS), de l'adrénoleucodystrophie néonatale (NALD), de la dysplasie oculo-déto-digitale avec anomalies de la substance blanche cérébrale, de la leucodystrophie orthochromatique avec glie pigmentée, du syndrome d'ovarioleucodystrophie, de la maladie de Pelizaeus Merzbacher (paraplégie spastique liée au sexe), de la maladie de Refsum, du syndrome de Sjögren-Larssen, de la leucodystrophie soudanophile, du syndrome de Van der Knaap (leucodystrophie vacuolante avec kystes sous-corticaux ou MLC), de la maladie de la substance blanche en voie de disparition (VWM) ou de l'ataxie infantile avec hypomyélinisation diffuse du système nerveux central (CACH), de la maladie de Refsum du nourrisson et de la leucoencéphalopathie avec atteinte du tronc cérébral et de la moelle épinière et élévation du lactate (LBSL)/ leucoencéphalopathie DARS2.

4. Complexe de dendrimère destiné à être utilisé dans un procédé selon l'une quelconque des revendications 1 à 3, dans lequel le sujet est un enfant entre la naissance et 18 ans.

5. Complexe de dendrimère destiné à être utilisé dans un procédé selon l'une quelconque des revendications 1 à 4, dans lequel les dendrimères de PAMAM sont des dendrimères de PAMAM de génération 6.

6. Complexe de dendrimère destiné à être utilisé dans un procédé selon l'une quelconque des revendications 1 à 5, dans lequel les dendrimères sont conjugués à l'agent thérapeutique en une quantité efficace pour réduire ou inhiber la β-oxydation peroxysomale, la sécrétion de glutamate, une ou plusieurs pro-cytokines inflammatoires, ou toute combinaison de celles-ci dans la microglie activée.

7. Complexe de dendrimère destiné à être utilisé dans un procédé selon l'une quelconque des revendications 1 à 6, dans lequel les dendrimères sont conjugués à un ou plusieurs agents thérapeutiques en une quantité efficace pour réduire ou soulager autrement le stress oxydatif, la neuroinflammation, la production d'acides gras à longue chaîne, la perte de la fonction motrice, ou une combinaison de ceux-ci ; pour favoriser, augmenter ou améliorer la prolifération des peroxysomes, l'élimination des acides gras à très longue chaîne, la fonction motrice, l'expression d'ABCD2, les enzymes mutées ou déficientes dans les troubles peroxysomaux, ou une combinaison de celles-ci.

8. Complexe de dendrimère destiné à être utilisé dans un procédé selon l'une quelconque des revendications 1 à 7, dans lequel l'agent thérapeutique est :
un anti-inflammatoire ou un antioxydant ; éventuellement
dans lequel l'anti-inflammatoire est choisi dans le groupe constitué d'agents anti-inflammatoires stéroïdiens, d'agents anti-inflammatoires non stéroïdiens et d'agents anti-inflammatoires à base d'or ;
éventuellement encore
dans lequel l'anti-inflammatoire est VBP15 ;
une copie de type sauvage d'une enzyme qui est par ailleurs mutée ou déficiente dans un trouble peroxysomal ou une leucodystrophie ou un acide nucléique codant pour l'enzyme ; éventuellement
dans lequel l'enzyme est la galactosylcéramidase (GALC), l'aspartoacylase (ASPA) ou l'arylsulfatase A (ARSA) ;
une hormone thyroïdienne ou un thyromimétique ; éventuellement
dans lequel l'hormone thyroïdienne est une triiodothyronine naturelle ou synthétique (T3), sa prohormone thyroxine (T4), ou un mélange de celles-ci ; ou
dans lequel le thyromimétique est le sobétirome ;
le butyrate de 4-phényle, le benzafibrate, la N-acétylcystéine, la pioglitazone ou la vitamine E ; un agent qui améliore l'homéostasie redox et/ou la respiration mitochondriale ; un agent qui réduit ou inverse la défaillance bioénergétique, la dégénérescence axonale et/ou les handicaps locomoteurs associés, ou une combinaison de ceux-ci ; éventuellement
dans lequel l'agent est le resvératrol.

9. Complexe de dendrimère destiné à être utilisé dans un procédé selon l'une quelconque des revendications 1 à 8, dans lequel le dendrimère est conjugué à deux agents thérapeutiques différents ; éventuellement
dans lequel le premier agent thérapeutique est un anti-inflammatoire ou un antioxydant ; et
dans lequel le second agent thérapeutique est choisi dans le groupe constitué de la N-acétylcystéine, du butyrate de 4-phényle, du bézafibrate, de l'hormone thyroïdienne (T3), du sobétirome, de la pioglitazone, du resvératrol, du VBP15, de la vitamine E, de l'acide érucique, de la biotine, de la coenzyme Q10, de la clémastine, de la galactosylcéramidase (GALC) et de l'arylsulfatase A (ARSA).

10. Complexe de dendrimère destiné à être utilisé dans un procédé selon l'une quelconque des revendications 1 à 9, dans lequel l'agent thérapeutiquement actif est destiné à localiser et à cibler des neurones spinaux positifs à la bêta-tubuline de classe III spécifique aux neurones (TUJ-1).

11. Complexe de dendrimère destiné à être utilisé dans un procédé selon l'une quelconque des revendications 1 à 10, dans lequel le complexe de dendrimère est formulé dans une suspension, une émulsion ou une solution ; et/ou est administré au sujet dans une période de temps choisie dans le groupe constitué de : tous les deux jours, tous les trois jours, tous les 4 jours, de manière hebdomadaire, bihebdomadaire, mensuelle et bimensuelle.

12. Composition de dendrimère comprenant des dendrimères complexés, liés par covalence ou dispersés de manière intramoléculaire ou encapsulés avec un agent thérapeutique, dans laquelle les dendrimères sont des dendrimères de poly(amidoamine) (PAMAM) à terminaison hydroxyle de génération 4 à 6 et l'agent thérapeutique est la coenzyme Q10, la clémastine, VBP15, des copies de type sauvage de galactosylcéramidase (GALC) ou d'arylsulfatase A (ARSA) ou un acide nucléique codant pour GALC ou ARSA, une hormone thyroïdienne, un thyromimétique, le butyrate de 4-phényle, l'acide érucique, la pioglitazone, le benzafibrate ou toute combinaison de ceux-ci.

13. Composition de dendrimère selon la revendication 12, dans laquelle l'hormone thyroïdienne est la triiodothyronine (T3) naturelle ou synthétique, sa prohormone thyroxine (T4), ou un mélange de celles-ci.

14. Composition de dendrimère selon la revendication 12, dans laquelle le thyromimétique est le sobétirome.
